# FASCICULE DE BREVET EUROPEEN

(11) **EP 1 651 596 B1**
(45) Date de publication et mention de la délivrance du brevet: **13.01.2010**
(21) Numéro de dépôt: 04786279.2
(22) Date de dépôt: 06.08.2004
(51) Int. Cl.: C07C 323/58, C07C 323/66, C07C 323/61, C07F 9/40, C07F 9/38

(54) **DERIVES DE 4,4'-DITHIOBIS-(3-AMINOBUTANE-1-SULFONATES) NOUVEAUX ET COMPOSITIONS LES CONTENANT**
NEUE DERIVATE VON 4,4'-DITHIOBIS-(3-AMINOBUTAN-1-SULFONATEN) UND DIESE ENTHALTENDE ZUSAMMENSETZUNGEN
NOVEL DERIVATIVES OF 4,4'-DITHIOBIS-(3-AMINOBUTANE-1-SULFONATES) AND COMPOSITIONS COMPRISING THE SAME

(30) Priorité: 06.08.2003 FR 0309700
(43) Date de publication de la demande: 03.05.2006
(73) Titulaire: INSTITUT NATIONAL DE LA SANTE ET DE LA RECHERCHE MEDICALE (INSERM), 75654 Paris Cédex 13 (FR); UNIVERSITE PARIS DESCARTES, 75270 Paris Cedex 06 (FR); CENTRE NATIONAL DE LA RECHERCHE SCIENTIFIQUE (CNRS), 75794 Paris Cedex 16 (FR)
(72) Inventeur: ROQUES, Bernard, Pierre, F-75014 Paris (FR); INGUIMBERT, Nicolas, F-94230 Cachan (FR); FOURNIE-ZALUSKI, Marie-Claude, F-75011 Paris (FR); CORVOL, Pierre, F-75007 Paris (FR); LLORENS-CORTES, Catherine, F-91440 Bures sur Yvette (FR)
(74) Mandataire: Ulmann, Catherine Claire
(86) Numéro de dépôt international: PCT/FR2004/002106
(87) Numéro de publication internationale: WO 2005/014535

(56) Documents cités:
- CHAUVEL ET AL: "INVESTIGATION OF THE ACTIVE SITE OF AMINOPEPTIDASE A USING A SERIESOF NEW THIOL-CONTAINING INHIBITORS" JOURNAL OF MEDICINAL CHEMISTRY, AMERICAN CHEMICAL SOCIETY. WASHINGTON, US, vol. 37, no. 9, 1994, pages 1339-1346, XP002068834 ISSN: 0022-2623
- CHAUVEL E N ET AL: "DIFFERENTIAL INHIBITION OF AMINOPEPTIDASE A AND AMINOPEPTIDASE N BYNEW BETA-AMINO THIOLS" JOURNAL OF MEDICINAL CHEMISTRY, AMERICAN CHEMICAL SOCIETY. WASHINGTON, US, vol. 37, no. 18, 1994, pages 2950-2957, XP002068832 ISSN: 0022-2623
- MARTIN L ET AL: "Beta-amino-thiols inhibit the zinc metallopeptidase activity of tetanus toxin light chain" JOURNAL OF MEDICINAL CHEMISTRY, AMERICAN CHEMICAL SOCIETY. WASHINGTON, US, vol. 41, no. 18, 27 août 1998 (1998-08-27), pages 3450-3460, XP002092584 ISSN: 0022-2623

## Description

La présente invention a pour objet des composés nouveaux, des procédés pour la préparation de ces composés, des formulations pharmaceutiques comprenant ces composés, et l'utilisation de ces composés pour la préparation de médicaments. La présente invention concerne en particulier des composés qui sont utiles dans le traitement et la prévention de l'hypertension artérielle primaire et secondaire, d'un ictus, de l'ischémie myocardique, de l'insuffisance cardiaque et de l'insuffisance rénale, de l'infarctus du myocarde, d'une maladie vasculaire périphérique, de la protéinurie diabétique, du syndrome X et du glaucome.

L'hypertension artérielle est une affection dont les causes restent généralement inconnues. Des facteurs extrinsèques qui peuvent participer comprennent l'obésité, un mode de vie sédentaire, l'absorption excessive d'alcool ou de sel et le stress. Des facteurs intrinsèques suggérés en tant que facteurs jouant un rôle comprennent la rétention de fluide, l'activité du système nerveux sympathique et la constriction des vaisseaux sanguins. L'hypertension artérielle peut contribuer directement ou indirectement à des maladies du coeur, du système vasculaire périphérique et cérébral, du cerveau, de l'oeil et du rein.

Le traitement de l'hypertension artérielle comprend l'utilisation d'agents diurétiques, d'agents de blocage adrénergique, d'inhibiteurs de l'enzyme de conversion d'angiotensine, d'antagonistes des récepteurs d'angiotensine, d'antagonistes du calcium et de vasodilatateurs directs. Néanmoins un certain nombre de patients restent réfractaires à tous les traitements, aggravant ainsi le risque de maladies diverses liées à leur hypertension et en particulier l'installation d'une insuffisance cardiaque chronique. Il est donc souhaitable d'identifier des composés nouveaux pour le traitement de l'hypertension artérielle.

Les présents inventeurs ont identifié des composés nouveaux qui sont efficaces dans la réduction de l'hypertension artérielle et qui, ainsi, sont utiles dans le traitement de l'hypertension artérielle et des maladies auxquelles elle contribue indirectement et directement.

Ces composés se comportent en particulier comme de puissants inhibiteurs de l'aminopeptidase A (également dénommée APA ou EC 3.4.11.7) qui est une métallopeptidase à zinc très conservée d'une espèce à l'autre, y compris l'homme. Il a été démontré que l'APA agissait sur la régulation centrale de la pression artérielle (A. Reaux et al., Proc. Natl. Acad.Sci. USA, 1999, 96, 13415-13420 et M.C. Fournié-Zaluski et al., Proc Natl Acad Sci. USA 2004; 101, 7775-7780). La présente invention montre que de manière inattendue, l'introduction d'un groupe R₂ sur une structure non peptidique conduit à l'obtention de composés inhibiteurs de l'APA ayant une affinité et une sélectivité pour l'APA élevée, alors que ces composés n'ont pas d'activité vis-à-vis d'une autre aminopeptidase, l'aminopeptidase N (APN).

En conséquence, la présente invention comprend des composés de formule (1) : 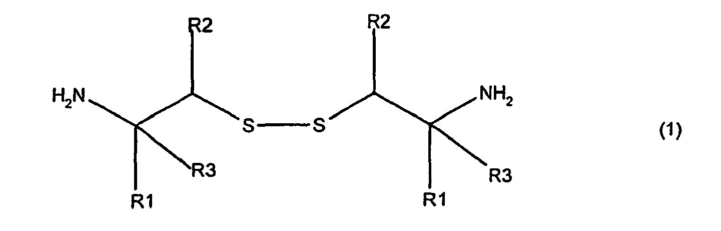 dans laquelle
chaque groupement R¹ est identique à l'autre groupement R¹ et représente :
- un groupe alkyle en C₁ à C₆, alcényle en C₂ à C₆ ou alcynyle en C₂ à C₆,
- un groupe (CH₂)ₙbenzyle dans lequel n est égal à 0 ou 1,
- un groupe (CH₂)ₘ(cycloalkyle en C₃ à C₆) dans lequel m est égal à 0 ou 1, chacun des groupes alkyle, alcényle, alcynyle, benzyle ou cycloalkyle étant substitué par un ou deux groupe(s) représenté(s) par le groupement A.

Le groupement A représente :
- un groupe carboxylate COOH ou COOR, R représentant un groupe alkyle en C₁ à C₆ ou CH²phényle ;
- un groupe sulfonate SO₃H ou SO₃R', R' représentant un groupe alkyle en C₁ à C₆ ou CH₂phényle ;
- un groupe phosphonate PO₃H₂ ou PO₃R₂"R"', R" et R"' représentant indépendamment H, un groupe alkyle en C₁ à C₆ ou CH₂phényle ; chaque groupement R² est identique à l'autre groupement R² et représente un groupe alkyle en C₁ à C₆, alcényle en C₂ à C₆ ou alcynyle en C₂ à C₆, chaque groupe alkyle, alcényle ou alcynyle étant libre ou substitué par le groupement B.

Le groupement B représente :
- un groupe carboxylate, COOH ou COOR', R' représentant un groupe alkyle en C₁ à C₆ ou CH₂phényle ;
- un groupe phényle libre ou substitué par un ou plusieurs radicaux choisis parmi un atome d'halogène, un radical hydroxyle éventuellement protégé, un groupe alkyle en C₁ à C₄, un groupe cyano, un groupe carboxyle libre, salifié, estérifié ou un groupe amide

Chaque groupement R³ est identique à l'autre groupement R³ et représente un atome d'hydrogène.

Des composés (1) préférés selon l'invention sont ceux dans lesquels R¹ est choisi entre des groupes alkyle en C₁ à C₆, alcényle en C₂ à C₆ et benzyle, chacun de ces groupes étant substitué par un ou deux groupe(s) représenté(s) par le groupement A et /ou dans lesquels R² est choisi entre un groupe alkyle en C₁ à C₆ et un groupe alcényle en C₂ à C₆, chacun de ces groupes pouvant être substitué par un ou deux groupe(s) représenté(s) par le groupement B.

D'autres composés (1) préférés selon l'invention sont ceux dans lesquels R¹ représente un groupe éthyle substitué par un groupe sulfonique, phosphonique ou carboxylique, libre, salifié ou estérifié et R² représente un groupe éthyle substitué par un groupe phényle éventuellement substitué.

Un composé (1) particulièrement préféré est l'acide 4,4'-dithiobis-(3,3'-amino-6,6'-phényl-1-1'-hexane sulfonique), et notamment l'acide 4(S),4'(S),3(S), 3'(S)-4'-dithiobis-(3,3'-amino-6,6'-phényl-1-1'-hexane sulfonique).
Dans un autre aspect, la présente invention propose des compositions pharmaceutiques comprenant un composé de la présente invention, de préférence en association avec un diluant ou support pharmaceutiquement acceptable.

Dans un autre aspect, la présente invention propose un composé de la présente invention destiné à être utilisé en thérapeutique, et en particulier en médecine humaine.

L'invention concerne également l'utilisation d'un composé de formule (1) en tant qu'inhibiteur sélectif à l'égard de l'aminopeptidase A.

Dans un autre aspect, la présente invention propose l'utilisation d'un composé de la présente invention pour la production d'un médicament destiné au traitement de l'hypertension artérielle et de maladies indirectement et directement liées.

Ces maladies comprennent des maladies du coeur, du système vasculaire périphérique et cérébral, du cerveau, de l'oeil et du rein. En particulier, les maladies comprennent l'hypertension artérielle primaire et secondaire, un ictus, l'ischémie myocardique, l'insuffisance cardiaque et l'insuffisance rénale, l'infarctus du myocarde, une maladie vasculaire périphérique, la protéinurie diabétique, le syndrome X, le glaucome, les maladies neurodégénératives et les troubles de la mémoire. Par ailleurs l'hypertension, en particulier centrale, est éventuellement repliée à une hyperexpression vasculaire de l'APA. Celle-ci s'accroît encore dans les tumeurs. De ce fait, les composés de la présente invention pourraient avoir un potentiel thérapeutique dans le cadre des pathologies ischémiques ou tumorales (Marchio S, et al., Cancer Cell, 2004, 5:151-162).

L'invention concerne donc également l'utilisation d'un composé de formule (1) pour la préparation d'un médicament destiné au traitement de pathologies ischémiques ou tumorales dans lesquelles l'APA est impliquée:

Telle qu'elle est utilisée dans le présent mémoire, l'expression "composé de la présente invention" désigne un composé de formule (1) ou un de ses sels ou produit de solvatation pharmaceutiquement acceptable.

L'expression "alkyle en C₁ à C₆", telle qu'elle est utilisée dans le présent mémoire, désigne un groupe hydrocarboné à chaîne droite ou ramifiée contenant 1 à 6 atomes de carbone. Des exemples de groupes alkyle, de la manière utilisée dans le présent mémoire, comprennent, mais à titre non limitatif, les groupes méthyle, éthyle, propyle, butyle, isopropyle, n-butyle et tertiobutyle.

L'expression "alcényle en C₂ à C₆", telle qu'elle est utilisée dans le présent mémoire, désigne un groupe hydrocarboné à chaîne droite ou ramifiée ayant 1 à 6 atomes de carbone, contenant une ou plusieurs doubles liaisons. Des exemples de groupes alcényle, de la manière utilisée dans le présent mémoire, comprennent, mais à titre non limitatif, le groupe vinyle et des groupes similaires.

L'expression "alcynyle en C₂ à C₆", telle qu'elle est utilisée dans le présent mémoire, désigne un groupe hydrocarboné à chaîne droite ou ramifiée ayant 1 à 6 atomes de carbone, contenant une ou plusieurs triples liaisons. Un exemple de groupe alcynyle, de la manière utilisée dans le présent mémoire, comprend, mais à titre non limitatif, le groupe éthynyle.

L'expression "cycloalkyle en C₃ à C₆" désigne un noyau carboné cyclique non aromatique ayant 3 à 6 atomes de carbone. Ce noyau peut contenir facultativement jusqu'à 2 doubles liaisons carbone - carbone. Les groupes cycloalkyle comprennent, à titre d'exemple mais non à titre limitatif, les groupes cyclopentyle et cyctohexyle.

De préférence. R¹ est choisi entre des groupes alkyle en C₁ à C₆, alcényle en C₂ à C₆ et benzyle substitués avec un ou deux groupes représentés par le groupement A répondant à la définition précitée.

De préférence, R² est choisi entre des groupes alkyle en C₁ à C₆ et alcényle en C₂ à C₆, chaque groupe alkyle ou alcényle étant facultativement substitué avec un ou plusieurs groupes représentés par le groupement B défini ci-dessus.

Bien que les groupes préférés pour chaque variable aient été généralement énumérés ci-dessus séparément pour chaque variable, des composés appréciés de la présente invention comprennent ceux dans lesquels plusieurs variables ou chaque variable dans la formule (I) sont (est) choisie(s) parmi les groupes appréciés, plus appréciés ou préférés pour chaque variable. En conséquence, la présente invention est destinée à comprendre toutes les associations de groupes appréciés, plus appréciés et préférés.

L'homme de l'art reconnaîtra que des stéréocentres existent dans les composés de formule (I). En conséquence, la présente invention comprend tous les stéréo-isomères et isomères géométriques possibles de la formule (I) et comprend non seulement des composés racémiques mais également les isomères optiquement actifs. Lorsqu'un composé de formule (I) est désiré sous forme d'un énantiomère unique, il peut être obtenu par résolution du produit final ou par synthèse stéréospécifique à partir de la matière de départ isomériquement pure ou bien de n'importe quel intermédiaire convenable. La résolution du produit final, d'un intermédiaire ou d'une matière de départ peut être effectuée par n'importe quel procédé convenable connu dans ce domaine. Voir, par exemple, Stereochemistry of Carbon Compounds par E. L. Eliel (Mcgraw Hill, 1962) et Tables of Resolving Agents par S. H. Wilen. En outre, dans les cas où des formes tautomères des composés de formule (I) sont possibles, la présente invention est destinée à comprendre toutes les formes tautomères des composés.

Le spécialiste de la chimie organique notera que de nombreux composés organiques peuvent former des complexes avec des solvants dans lesquels ils ont été amenés à réagir ou à partir desquels ils sont précipités ou cristallisés. Ces complexes sont connus sous le nom de "produits de solvatation". Par exemple, un complexe avec l'eau est connu sous le nom de "hydrate". Les produits de solvatation du composé de formule (I) entrent dans le cadre de la présente invention.

Le spécialiste de la chimie organique notera également que de nombreux composés organiques peuvent exister sous plus d'une forme cristalline. Par exemple, la forme cristalline peut varier d'un produit de solvatation à l'autre. Ainsi, toutes les formes cristallines des composés de formule (I) ou de leurs produits de solvatation pharmaceutiquement acceptables sont incluses dans le cadre de la présente invention.

L'homme de l'art notera également que les composés de la présente invention peuvent être également utilisés sous forme d'un de leur sel ou produit de solvatation pharmaceutiquement acceptable. Les sels physiologiquement acceptables des composés de formule (I) comprennent des sels classiques formés à partir d'acides ou de bases inorganiques ou organiques pharmaceutiquement acceptables ainsi que des sels d'addition d'ammonium quaternaire. Des exemples plus spécifiques de sels d'acides convenables comprennent les sels formés avec les acides chlorhydrique, bromhydrique, sulfurique, phosphorique, nitrique, perchlorique, fumarique, acétique, propionique, succinique, glycolique, formique, lactique, maléique, tartrique, citrique, palmoïque, malonique, hydroxymaléique, phénylacétique, glutamique, benzoïque, salicylique, fumarique, toluènesulfonique, méthane-sulfonique, naphtalène-2-sulfonique, benzènesulfonique, hydroxynaphtoïque, iodhydrique, malique, stéroïque, tannique, etc. D'autres acides tels que l'acide oxalique, bien que n'étant pas en eux-mêmes pharmaceutiquement acceptables, peuvent être utiles dans la préparation de sels utiles comme intermédiaires dans l'obtention des composés de la présente invention et de leurs sels pharmaceutiquement acceptables. Des exemples plus spécifiques de sels basiques convenables comprennent les sels de sodium, de lithium, de potassium, de magnésium, d'aluminium, de calcium, de zinc, de N,N'-dibenzyléthylène-diamine, de chloroprocaïne, de choline, de diéthanolamine, d'éthylènediamine, de N-méthylglucamine et de procaïne. Des références ci-après à un composé conforme à la présente invention concernent à la fois les composés de formule (I) et leurs sels et produits de solvatation pharmaceutiquement acceptables.

Les composés de la présente invention et leurs dérivés pharmaceutiquement acceptables sont administrés convenablement sous forme de compositions pharmaceutiques. Ces compositions peuvent être présentées convenablement à des fins d'utilisation de manière classique en mélange avec un ou plusieurs supports ou excipients physiologiquement acceptables. Le ou les supports doivent être "acceptables" en ce sens qu'ils doivent être compatibles avec les autres ingrédients de la formulation et ils ne doivent pas être néfastes pour le sujet les recevant.

Bien qu'il soit possible d'administrer thérapeutiquement les composés de la présente invention sous forme de la substance chimique brute, il est préférable de présenter l'ingrédient actif sous forme d'une formulation pharmaceutique.

En conséquence, la présente invention propose en outre une formulation pharmaceutique comprenant un composé de formule (I) ou un de ses sels ou produit de solvatation pharmaceutiquement acceptable en association avec un ou plusieurs supports pharmaceutiquement acceptables et, facultativement, d'autres ingrédients thérapeutiques et/ou prophylactiques.

Les formulations comprennent celles convenables pour l'administration orale, parentérale (y compris sous-cutanée, par exemple par injection ou au moyen d'un comprimé à dépôt, intradermique, intrathécal, intramusculaire, par exemple par dépôt, et intraveineuse), rectale et topique (y compris dermique, buccale et sublinguale) ou sous une forme convenable pour l'administration par inhalation ou insufflation, bien que la voie convenant le mieux puisse dépendre, par exemple, de l'état et de l'affection du receveur. Les formulations peuvent être présentées convenablement sous une forme posologique unitaire et peuvent être préparées par n'importe lequel des procédés bien connus dans le domaine de la pharmacie. Tous les procédés comprennent l'étape consistant à mettre en association les composés ("ingrédients actifs") avec le support qui comprend un ou plusieurs ingrédients accessoires. En général, les formulations sont préparées en mettant uniformément et intimement en association l'ingrédient actif avec des véhicules liquides ou des supports solides finement divisés ou bien avec ces deux types de supports et ensuite, si nécessaire, en façonnant le produit en la formulation désirée.

Les formulations convenables pour l'administration orale peuvent être présentées sous forme d'unités discrètes telles que des capsules, cachets ou comprimés (par exemple des comprimés à mâcher, en particulier pour une administration pédiatrique), chacun contenant une quantité prédéterminée de l'ingrédient actif ; sous forme d'une poudre ou de granules ; sous forme d'une solution ou d'une suspension dans un liquide aqueux ou un liquide non aqueux ; ou sous forme d'une émulsion liquide huile-dans-eau ou d'une émulsion liquide eau-dans-huile. L'ingrédient actif peut également être présenté sous forme d'un bol, d'un électuaire ou d'une pâte.

Un comprimé peut être préparé par compression ou moulage, facultativement avec un ou plusieurs ingrédients accessoires. Des comprimés produits par compression peuvent être préparés en comprimant dans une machine convenable l'ingrédient actif sous une forme à écoulement libre telle qu'une poudre ou des granules, facultativement en mélange avec d'autres excipients classiques tels que des liants (par exemple un sirop, la gomme arabique, la gélatine, le sorbitol, la gomme adragante, un mucilage d'amidon, la polyvinylpyrrolidone ou l'hydroxyméthylcellulose), des charges (par exemple le lactose, le saccharose, la cellulose microcristalline, l'amidon de maïs, le phosphate de calcium ou le sorbitol), des lubrifiants (par exemple le stéarate de magnésium, l'acide stéarique, le talc, le polyéthylèneglycol ou la silice), des agents de délitement (par exemple la fécule de pomme de terre ou le glycolate d'amidon sodique) ou des agents mouillants tels que le laurylsulfate de sodium. Des comprimés moulés peuvent être préparés en moulant dans une machine convenable un mélange du composé pulvérisé humidifié avec un diluant liquide inerte. Les comprimés peuvent être facultativement enrobés ou entaillés et peuvent être formulés de manière à provoquer la libération lente ou contrôlée de l'ingrédient actif qui s'y trouve. Les comprimés peuvent être enrobés par d'es procédés bien connus dans ce domaine.

En variante, les composés de la présente invention peuvent être incorporés à des préparations liquides orales telles que des suspensions, solutions ou émulsions aqueuses ou huileuses, des sirops ou élixirs, par exemple. En outre, des formulations contenant ces composés peuvent être présentées à l'état de produits secs destinés à une reconstitution avec de l'eau ou un autre véhicule convenable avant utilisation. Ces préparations liquides peuvent contenir des additifs classiques tels que des agents de mise en suspension, par exemple le sirop de sorbitol, la méthylcellulose, le glucose/sirop de sucre, la gélatine, l'hydroxyéthylcellulose, la carboxyméthylcellulose, un gel de stéarate d'aluminium ou des matières grasses comestibles hydrogénées ; des agents émulsionnants tels que la lécithine, le monooléate de sorbitanne ou la gomme arabique ; des véhicules non aqueux (qui peuvent comprendre des huiles comestibles) tels que l'huile d'amande, l'huile de coprah fractionnée, des esters huileux, le propylèneglycol ou l'alcool éthylique ; et des conservateurs tels que le p-hydroxybenzoate de méthyle ou de propyle ou l'acide sorbique. Ces préparations peuvent également être formulées à l'état de suppositoires, contenant par exemple des excipients classiques pour suppositoires tels que le beurre de cacao ou d'autres glycérides.

Les formulations pour l'administration parentérale comprennent des solutions injectables stériles aqueuses et non aqueuses qui peuvent contenir des antioxydants, des tampons, des agents bactériostatiques et des solutés qui rendent la formulation isotonique avec le sang du receveur choisi ; et des suspensions aqueuses et non aqueuses stériles qui peuvent comprendre des agents de mise en suspension et des agents épaississants. Les formulations peuvent être présentées dans des récipients à dose unique ou doses multiples, par exemple des ampoules et flacons clos hermétiquement, et peuvent être stockées à l'état séché par congélation (lyophilisé) nécessitant seulement l'addition d'un véhicule liquide stérile, par exemple de l'eau pour préparations injectables, immédiatement avant utilisation. Des solutions et suspensions injectables extemporanées peuvent être préparées à partir de poudres, granules et comprimés stériles du type décrit précédemment.

Les formulations pour l'administration rectale peuvent être présentées à l'état de suppositoires avec les supports usuels tels que le beurre de cacao, une graisse dure ou le polyéthylèneglycol.

Les formulations pour l'administration topique dans la cavité buccale, par exemple pour l'administration buccale ou sublinguale, comprennent des tablettes comprenant l'ingrédient actif dans un excipient aromatisé tel que la saccharose et la gomme arabique ou la gomme adragante, et des pastilles comprenant l'ingrédient actif dans un excipient tel que la gélatine et le glycérol ou le saccharose et la gomme arabique.

Pour l'administration topique à l'épiderme, les composés peuvent être formulés à l'état de crèmes, de gels, de pommades ou de lotions ou sous forme d'un timbre transdermique.

Les composés peuvent également être formulés à l'état de préparations pour dépôt. Ces formulations à longue durée d'action peuvent être administrées par implantation (par exemple par voie sous-cutanée ou intramusculaire) ou bien par injection intramusculaire. Ainsi, par exemple, les composés peuvent être formulés avec des matières polymères ou hydrophobes convenables (par exemple sous forme d'une émulsion dans une huile acceptable) ou des résines échangeuses d'ions, ou sous forme de dérivés très faiblement solubles, par exemple sous forme d'un sel très faiblement soluble.

Pour l'administration intranasale, les composés de la présente invention peuvent être utilisés, par exemple, sous forme d'un liquide d'atomisation, d'une poudre ou de gouttes.

Pour l'administration par inhalation, les composés conformes à la présente invention sont délivrés convenablement sous forme d'un aérosol émis par pulvérisation par un récipient sous pression ou un nébuliseur, au moyen d'un agent propulseur convenable, par exempte le 1,1,1,2-trifluoréthane (HFA 134A) et le 1,1,1,2,3,3,3-heptafluoropropane (HFA 227), le dioxyde de carbone ou un autre gaz convenable. Dans le cas d'un aérosol sous pression, la dose exacte peut être déterminée en installant une valve destinée à délivrer une quantité mesurée. Les capsules et cartouches constituées, par exemple, de gélatine, destinées à être utilisées dans un inhalateur ou insufflateur peuvent être formulées de manière à contenir un mélange de poudres constitué d'un composé de la présente invention et d'un excipient en poudre convenable tel que le lactose ou l'amidon.

En plus des ingrédients mentionnés particulièrement ci-dessus, les formulations peuvent comprendre d'autres agents classiques dans ce domaine en rapport avec le type de formulation en question ; par exemple, les formulations convenables pour l'administration orale peuvent comprendre des agents aromatisants.

L'homme de l'art notera qu'une référence dans le présent mémoire à un traitement s'étend à la prophylaxie ainsi qu'au traitement de maladies ou symptômes établis. En outre, on notera que la quantité d'un composé de la présente invention requise pour l'utilisation dans un traitement varie en fonction de la nature de l'affection traitée et de l'âge et de l'état du patient et sera finalement laissée à la discrétion du médecin traitant ou vétérinaire. Cependant, en général, les doses utilisées pour le traitement d'un patient humain adulte sont comprises habituellement dans l'intervalle de 0,02 à 5000 mg par jour, de préférence de 1 à 1500 mg par jour. La dose désirée peut être présentée convenablement en une dose unique ou de manière fractionnée en plusieurs doses administrées à des intervalles appropriés, par exemple sous forme de deux, trois, quatre ou plus de quatre doses secondaires par jour. Les formulations conformes à la présente invention peuvent contenir 0,1 à 99 % de l'ingrédient actif, convenablement 30 à 95 % pour les comprimés et capsules et 3 à 50 % pour les préparations liquides.

Le composé de formule (I) destiné à être utilisé dans la présente invention peut être utilisé en association avec un ou plusieurs autres agents thérapeutiques, par exemple des antagonistes des récepteurs bêta-adrénergiques, des agents de blocage du canal calcium, des diurétiques du type thiazide, des antagonistes des récepteurs d'angiotensine et des inhibiteurs de l'enzyme de conversion d'angiotensine. Ainsi, la présente invention propose dans .un aspect supplémentaire l'utilisation d'une association comprenant un composé de formule (I) et un agent thérapeutique supplémentaire dans le traitement de l'hypertension actérielle.

Lorsque les composés de formule (I) sont utilisés en association avec d'autres agents thérapeutiques, les composés peuvent être administrés successivement ou simultanément par n'importe quelle voie convenable.

Les associations mentionnées ci-dessus peuvent être présentées convenablement à des fins d'utilisation sous forme d'une formulation pharmaceutique et, ainsi, des formulations pharmaceutiques comprenant une association répondant à la définition précitée conjointement de manière optimale avec un support ou excipient pharmaceutiquement acceptable constituent un aspect supplémentaire de la présente invention. Les différents constituants de ces associations peuvent être administrés successivement ou simultanément dans des formulations pharmaceutiques séparées ou combinées.

Lorsqu'ils sont combinés dans la même formulation, on notera que les deux composés doivent être stables et compatibles l'un avec l'autre et les autres constituants de la formulation et peuvent être formulés pour l'administration. Lorsqu'ils sont formulés séparément, ils peuvent être fournis dans n'importe quelle formulation convenable, commodément d'une manière connue pour de tels composés dans ce domaine.

Lorsqu'un composé de formule (I) est utilisé en association avec un second agent thérapeutique actif contre la même maladie, la dose de chaque composé peut différer de celle administrée lorsque le composé est utilisé seul. Les doses appropriées seront aisément déterminées par l'homme de l'art.

Les composés de formule (1) dans laquelle R¹ représente un groupe alkyle substitué par un groupement A qui est un groupe SO₃H ou SO₃R' et dans laquelle R² représente notamment un groupe alkyle substitué par un groupe B qui peut être un groupe phényle libre ou substitué peuvent être préparés par les procédés suivants, les procédés A et B étant préférentiellement utilisés.

Ces procédés décrivent notamment la préparation de monomères intermédiaires pour la synthèse des composés disulfures (ou composés dimères) de formule (1), qui peuvent qui peut être aisément obtenus par oxydation à l'iode.

Ces composés disulfures sont des composés précurseurs ou "prodrugs" dont la structure dimère facilite le passage de la barrière hémato-encéphalique (BHE). Le monomère, qui est le composé actif, est ensuite libéré par un processus physiologique (M.C. Fournié-Zaluski et al., Proc Natl Acad Sci. USA 2004; 101, 7775-7780).

Dans le procédé A, une réaction d'oléfination permet la transformation du triméthylester de la N-(benzyloxycarbonyl)-α-phosphonoglycine en déhydroaminoacide **1** selon la méthode de U. Schmidt, H. Griesser, V. Leitenberger, A. Lieberknecht, R. Mangold, R. Meyer, B. Rield. Synthesis, 1992, 487-490. Le composé **1** subit une réaction de Michael pour conduire à une cystéine β-substituée **2** avec un rendement global de 30 à 60%. Une procédure « one-pot » permet l'obtention d'un sulfonate α,β -insaturé 3 ou d'un phosphonate ou d'un carboxylate α,β -insaturé selon la méthode décrite par : C. David et al., Tetrahedron, 2000, 56, 209-215. La réduction du composé **3** par le borohydrure de sodium donne le composé **4**. La déprotection du composé **4** au reflux de l'acide trifluoroacétique en présence d'anisole permet après précipitation d'isoler le composé **5**, qui peut être aisément transformé en disulfure (composé **5 dimère**) par oxydation à l'iode.

### Procédé A

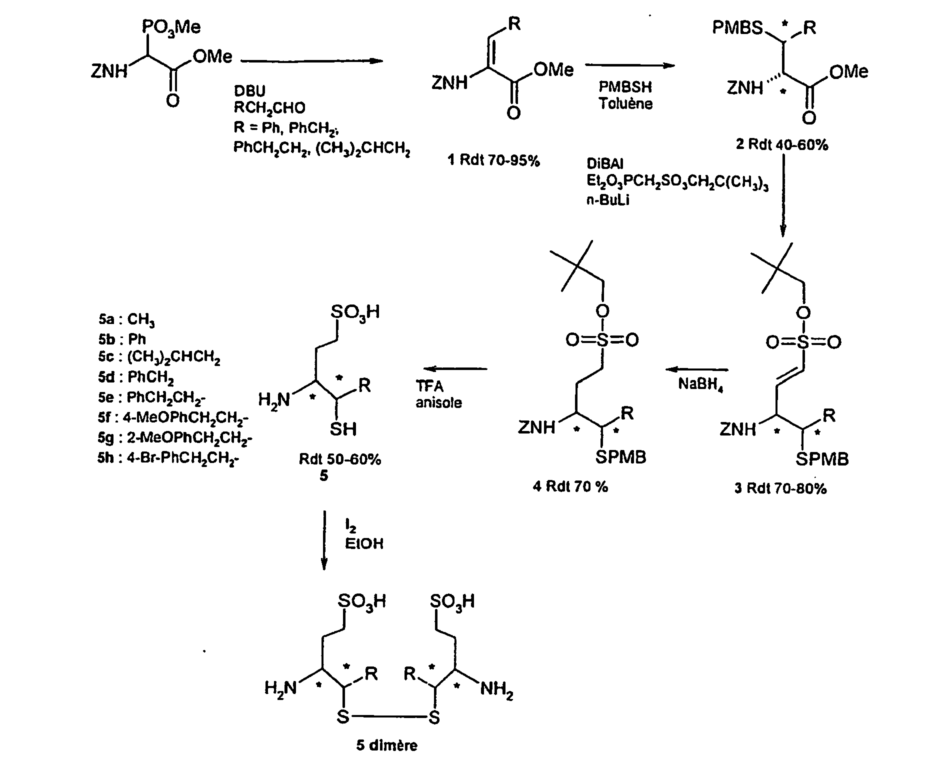

Z représente un groupe protecteur convenable, par exemple un groupe benzyloxycarbonyle.

Dans le but de déterminer les préférences stéréochimiques des composés **5**, une synthèse stéréosélective des quatre stéréo-isomères a été effectuée. Cette synthèse s'appuie sur l'obtention stéréosélective de cystéines β-substituées **2** selon la méthode de : C. Xiong, et al., J. Org. Chem., 2002, 67, 3514-3517 .

Ces synthèses sont décrites dans les schémas des procédés B1-B4 ci-après.

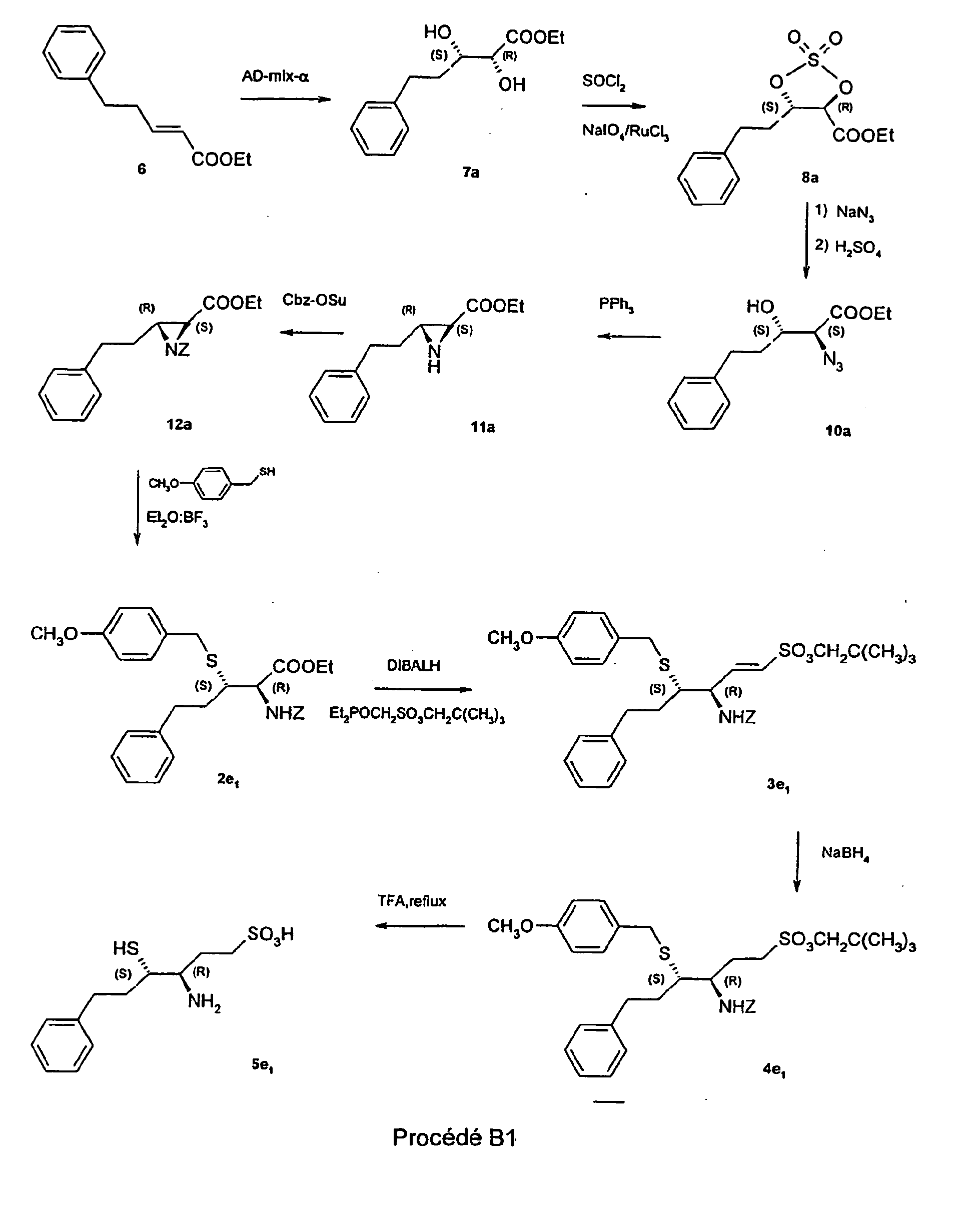

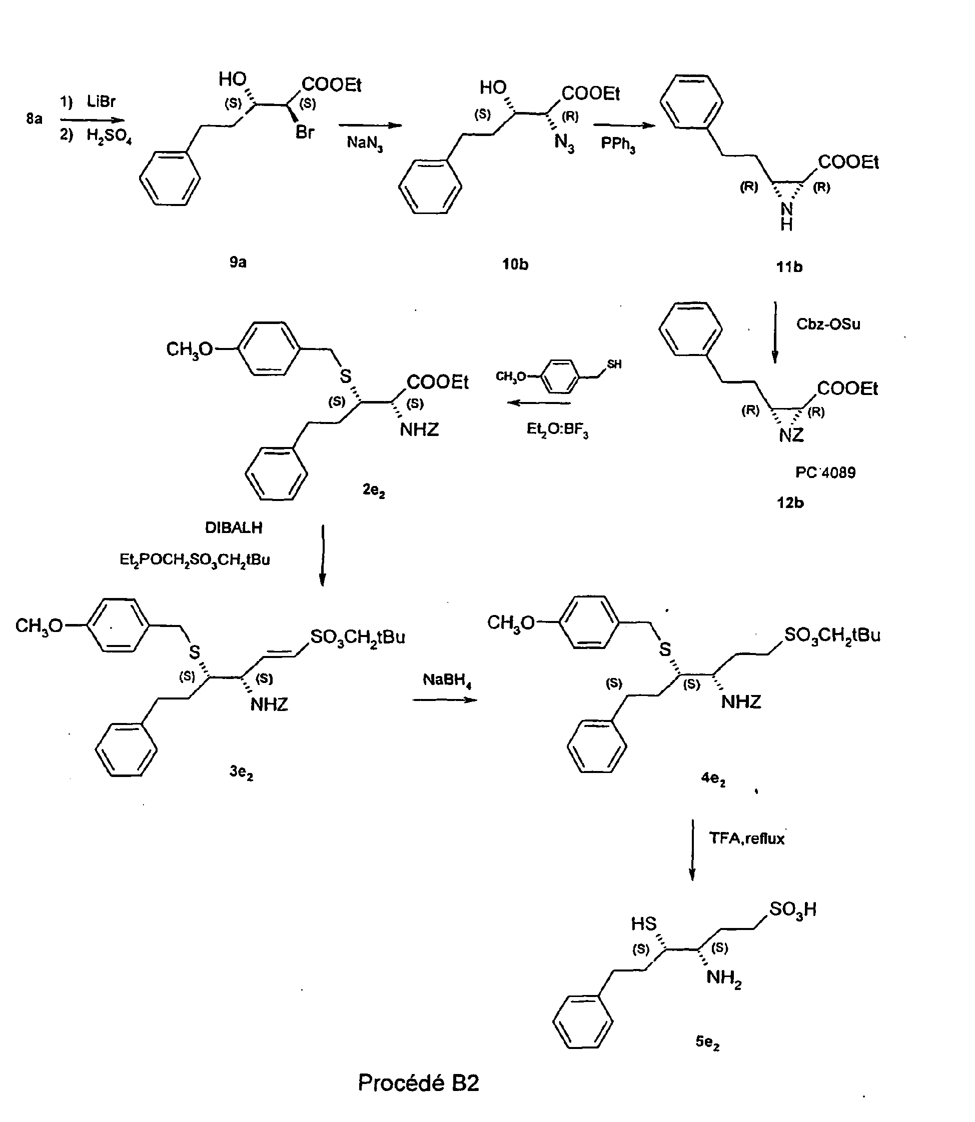

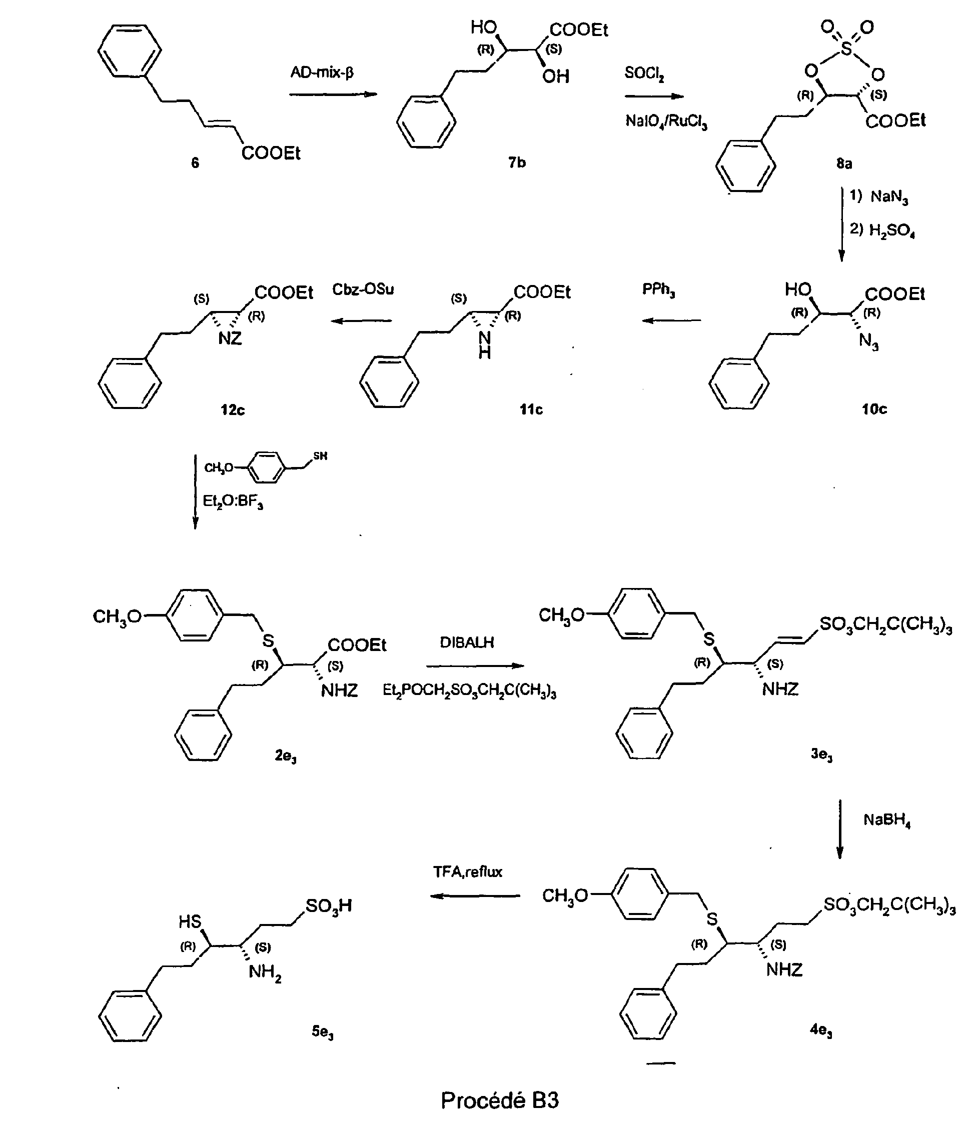

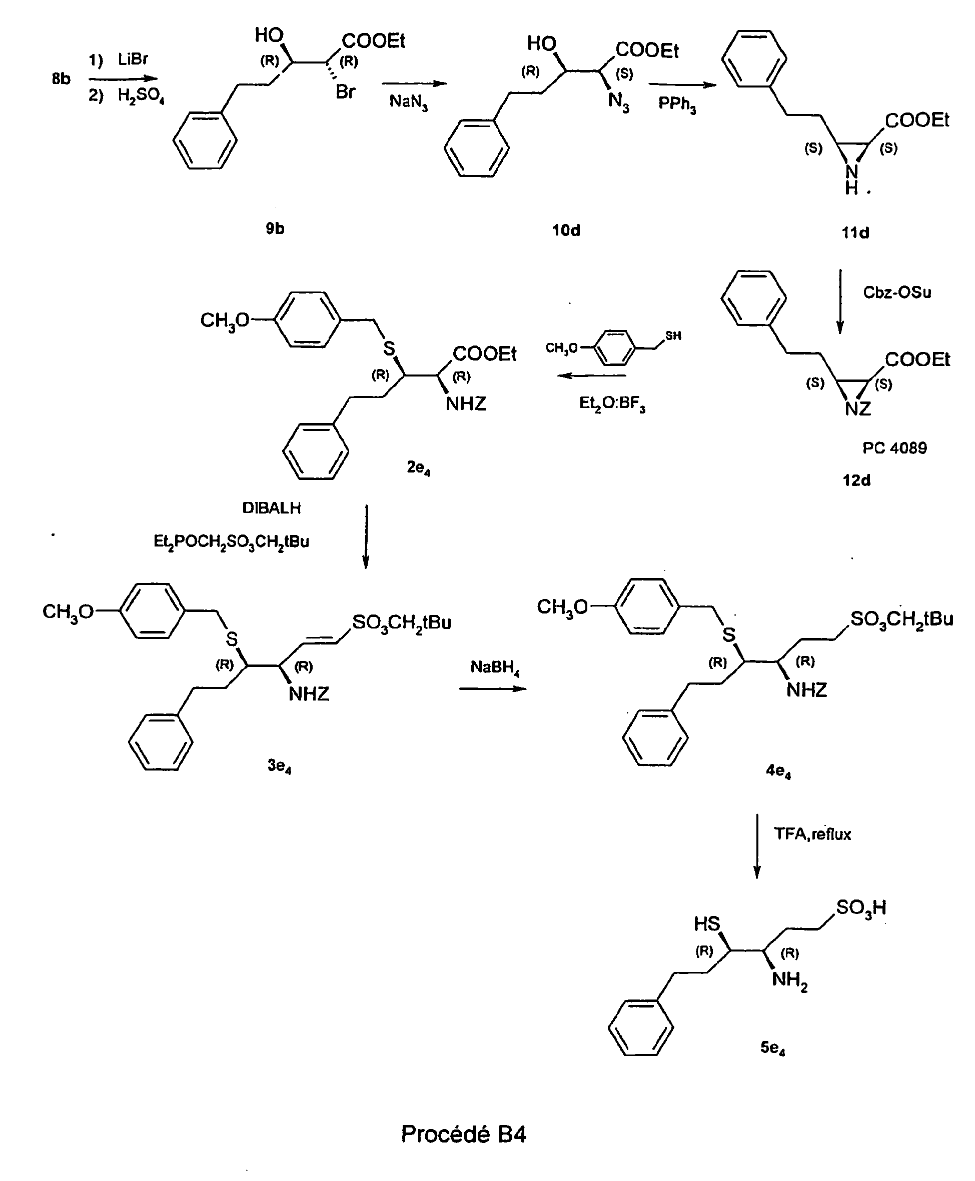

D'autres composés de formule (1) peuvent être préparés par des procédés analogues aux procédés ci-dessus et seront manifestes pour l'homme de l'art, comme par exemple le procédé illustré par le schéma 1 ci-après.

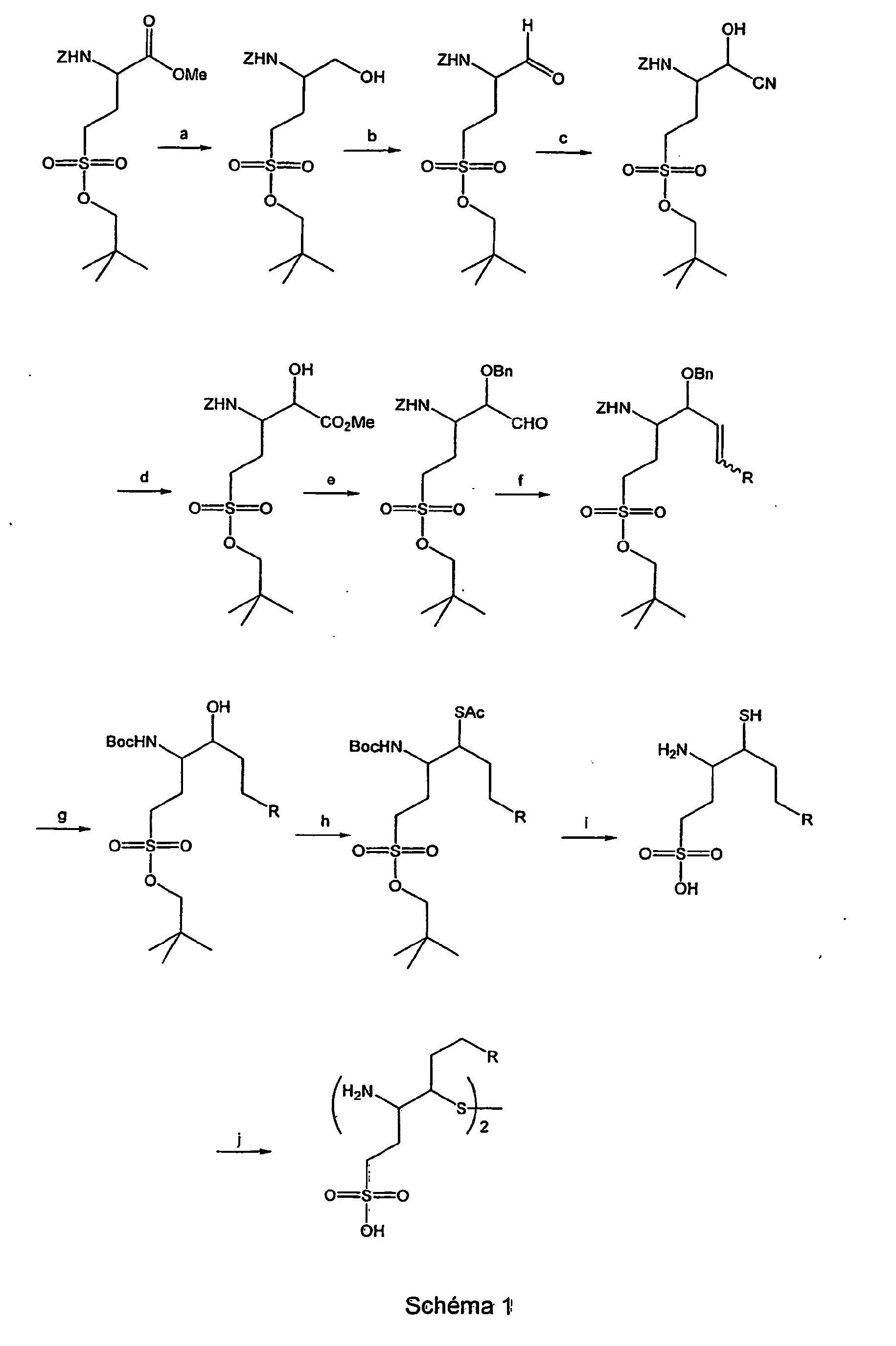

Dans le schéma 1, Z représente un groupe protecteur convenable, par exemple un groupe benzyloxycarbonyle et les lettres a à j représentent les conditions suivantes: a : NaBH₄, LiCl ; b : DMSO, (COCl₂)₂ ; c ; NaCN ; d ; HCl ; MeOH ; e : PhCh₂Br, NaH puis DIBAL ; f : RCH₂PPH₃Br, nBuLi ; g : H₂, Pd/C, Boc₂O ; h ; Mitsunobu ; i : HCI, reflux ; j : I₂, EtOH.

L'invention est illustrée de manière non limitative par les exemples suivants, dans lesquels la synthèse des monomères intermédiaires utilisables dans la synthèse des composés de formule (1) est décrite dans la partie « PREPARATIONS » et les numéros des composés se réfèrent aux procédés A et B décrits ci-dessus .

### PREPARATIONS

Dans les préparations 1 à 5 suivantes (procédé A), les lettres a à h se réfèrent aux composés pour lesquels la variable R a les définitions suivantes :
a R=CH3
b R=Ph
c R = (CH₃)₂CHCH₂-
d R = PhCH₂-
e R = PhCH₂CH₂-
f R = 4-MeOPhCH₂CH₂-
g R = 2-MeOPhCH₂CH₂-
h R = 4-Br-PhCH₂CH₂-
   avec Ph = phényle

Les préparations 6 à 16 se réfèrent aux procédés B1 à B4 décrits plus haut.

### Préparation 1 : Synthèse des composés 1

A une solution du triester méthylique de la N-benzyolxycarbonyl α-phosphonoglycine **12,** (5 mmol, 1,8 g) dans 10 ml de dichlorométhane on ajoute à 0°C, 0,76 ml (5 mmol) de diazabicycloundécène (DBU). Après 10 min on additionne l'aldéhyde (5 mmol). Le mélange réactionnel est agité à température ambiante une nuit. Le mélange réactionnel est dissous par 30 ml de dichlorométhane, on lave ensuite avec 2 x 10 ml, puis avec 2 x 10 ml d'une solution saturée de chlorure de sodium. La phase organique est séchée sur sulfate de sodium puis concentrée sous pression réduite, l'huile obtenue est purifiée par filtration sur silice (20 g) éluant cyclohexane/acétate d'éthyle 9/1.

On a obtenu de la même manière les composés 1a, 1b, 1c, 1d, 1e, 1f, 1g et 1h qui ont été caractérisés par leur spectre ¹H RMN dans CDCl₃ à 400MHz.

### Composé 1a :

### (2Z)-2-(N-benzyloxycarbonylamino)-but-2-ène-oate de méthyle

Rdt 72%. RMN ¹H (CDCl₃) : 1,85 (d, 3H, J = 7 Hz, CH₃), 3,75 (s, 3H, CH₃O), 5,15 (s, 2H, O-C**H₂**-C₆H₅), 6,2 (s, 1H, NH), 6,78 (q, 1H, J = 7 Hz, CH=C), 7,43 (m, 5H, O-CH₂-C₆**H₅**).

### Composé 1b

### (2Z)-2-(N-benzyloxycarbonylamino)-3-phényl-pro-2-ène-oate de méthyle

Rdt 55%. RMN ¹H (CDCl₃) 3,75 (s, 3H, CH₃O), 5,15 (s, 2H, O-C**H₂**-C₆H₅), 6,4 (s, 1H, NH), 7,43 (m, 5H, O-CH₂-C₆**H₅**), 7,52 (d, 1H, J = 3 Hz, CH=C).

### Composé 1c

### (2Z)-2-(N-benzyloxycarbonylamino)-5-méthyl-hex-2-éne-oate de méthyle

Rdt 95%. RMN ¹H (CDCl₃) : 0,95 (d, 6H, J = 7 Hz, CH-C**H₃**), 1,8 (hpt, 1H, J = 7Hz, C**H**-CH₃), 2,1 (t, 2H, J = 7 Hz, CH-C**H₂**), 3,75 (s, 3H, CH₃O), 5,15 (s, 2H, O-C**H**₂-C₆H₅), 6. 15 (s, 1H, NH), 6,78 (q, 1H, J = 7 Hz, CH=C), 7,43 (m, 5H, O-CH₂-C₆**H₅**).

### Composé 1d

### (2Z)1-2-(N-benzyloxycarbonylamino)-4-phényl-but-2-ène-oate de méthyle

Rdt 82%. RMN ¹H (CDCl₃) : 3,57 (d, 2H, J = 7 Hz, CH-C**H₂**-C₆H₅), 3,75 (s, 3H, CH₃O), 5,15 (s, 2H, O-C**H₂**-C₆H₅), 6,3 (s, 1H, NH), 6,78 (q, 1H, J = 7 Hz, CH=C), 7,2-7,3 (m, 5H, CH₂-C₆**H₅**), 7,43 (m, 5H, O-CH₂-C₆**H₅**).

### Composé 1e

### (2Z)-2-(N-benzyloxycarbonylamino)-5-phényl-pent-2-ène-oate d'éthyle

Rdt 100%. RMN ¹H (CDCl₃) : 2,52 (q, 2H, J = 7Hz, CH₂-CH₂-CH), 2,80 (t, 2H, J = 7Hz, C**H₂**-CH₂-CH), 3,75 (s, 3H, CH₃O), 5,15 (s, 2H, O-C**H₂-**C₆H₅), 6,1 (s, 1H, NH), 6,68 (t, 1H, J = 7 Hz, CH=C), 7,2 (m, 2H, CH₂-C₆**H₅**), 7,3 (m, 3H, CH₂-C₆**H₅**), 7,43 (m, 5H, O-CH₂-C₆**H₅**).

### Composé 1f

### (2Z)-2-(N-benzyloxycarbonylamino)-5-(4-méthoxy-phényl)-pent-2-ène-oate d'éthyle

Rdt 80%. RMN ¹H (CDCl₃) : 2,51 (q, 2H, J = 7Hz, CH₂-C**H₂**-CH), 2,72 (t, 2H, J = 7Hz, C**H₂**-CH₂-CH), 3,75 (s, 3H, CO₂CH₃), 3,8 (s, 3H, CH₃O),5,15 (s, 2H, O-C**H₂**-C₆H₅), 6,1 (s, 1H, NH), 6,65 (t, 1H, J = 7 Hz, CH=C), 6,8 (d, 2H, 4-CH₃O-C₆**H₄**), 7,1 (d, 2H, 4-CH₃O-C₆**H₄**), 7,3 - 7,4 (5H, m, CH₂-C₆**H₅**).

### Composé 1g

### (2Z)-2-(N-benzyloxycarbonylamino)-5-(2-méthoxy-phényl)-pent-2-ène-oate d'éthyle

Rdt 61%. RMN ¹H (CDCl₃) : 2,5 (m, 2H, CH₂-C**H₂**-CH), 2,75 (t, 2H, J = 7Hz, C**H₂**-CH₂-CH), 3,75 (s, 3H, CO₂CH₃), 3,8 (s, 3H, CH₃O),5,15 (s, 2H, O-C**H₂**-C₆H₅), 6,1 (s, 1H, NH), 6,7 (t, 1H, J = 7 Hz, CH=C), 6,85 (d, 1H, 2-CH₃OC₆**H₄**), 6,9 (dd, 1H, 2-CH₃O-C₆**H**₄), 7,1 (d, 1H, 2-CH₃O-C₆**H₄**), 7,2 (t, 1H, 2-CH₃O-C₆**H**₄), 7,3 - 7,4 (5H, m, CH₂-C₆**H₅**).

### Composé 1h

### (22)-2-(N-benzyloxycarbonylamino)-5-(-4-bromo-phényl)-pent-2-ène-oate d'éthyle

Rdt 41%. RMN ¹H (CDCl₃) : RMN ¹H (CDCl₃) : 2,51 (q, 2H, J = 7Hz, CH₂-C**H₂**-CH), 2,72 (t, 2H, J = 7Hz, C**H₂**-CH₂-CH), 3,75 (s, 3H, CO₂CH₃), 5,15 (s, 2H, O-C**H₂**-C₆H₅), 6,1 (s, 1H, NH), 6,65 (t, 1H, J = 7 Hz, CH=C), 7,0 (d, 2H, 4-Br-C₆**H**₄), 7,3 - 7,4 (5H, m, CH₂-C₆**H₅**), 7,5 (d, 2H, 4-Br-C₆**H**₄).

### Préparation 2 : Synthèse des composés 2

A une solution de **1** (5 mmol) et de 4-méthoxy-benzylmercaptan (10 mmol) dans 10 ml de toluène anhydre on ajoute 200 µl de pipéridine. Le mélange est porté à reflux 24 h sous argon. Le solvant est éliminé sous pression réduite. Après purification par chromatographie sur colonne (Eluant dichlorométhane) une huile est obtenue.

On a obtenu de la même manière les composés 2a, 2b, 2c, 2d, 2e, 2f, 2g et 2h qui ont été caractérisés par leur spectre ¹H RMN dans CDCl₃ à 400MHz.

### Composé 2a

### 2-Benzytoxycarbonylamino-3-(4-méthoxy-benzylsulfanyl)-butyrate de méthyle

Rdt 73%. HPLC (80%) : Tr = 5,2 et 5,4 min RMN ¹H (CDCl₃) : 1,2 et 1,3 (d, 3H, J = 7 Hz, CH₃), 3,1 et 3,3 (m, 1H, -CH-S), 3,6, 3,65, 3,7 et 3,75 (s, 8H, CO₂CH₃, S-CH2, CH₃O), 4,5 et 4,6 (dd, 1H, CH-CO₂), 5,15 (s, 2H, O-C**H₂-**C₆H₅), 5,4 et 5,55 (d, 1H, NH), 6,2 (s, 1H, NH), 6,78 (d, 2H, J = 7 Hz, S-C₆**H₄**), 7,2 (d, 2H, J = 7 Hz, S-C₆**H₄**), 7,43 (m, 5H, O-CH₂-C₆**H₅**).

### Composé 2b

### 2-Benzyloxycarbonylamino-3-(4-méthoxy-benzylsulfanyl)-3-phényl-propanoate de méthyle

Rdt 60%. HPLC (80%) : Tr = 6,7 min. RMN ¹H (CDCl₃): 3,5 et 3,6 (s, 2H, -CH₂-S), 3,6 (s, 3H, CH₃O), 3,75 (s, 3H, CO₂CH₃), 4,1 et 4,15 (d, 1H, J = 4 Hz, -CH-S), 4,65 et 4,85 (dd, ¹H, CH-CO₂), 5,15 (s, 2H, O-C**H₂**-C₆H₅), 5,2 et 5,45 (d, 1H, NH), 6,75 (d, 2H, J = 7 Hz, S-C₆**H₄**), 7,05 et 7.1 (d, 2H, J = 7 Hz, S-C₆**H₄**), 7,43 (m, 10H, O-CH₂-C₆**H₅** et CH-C₆**H₅**).

### Composé 2c

### 2-Benzyloxycarbonylamino-3-(4-méthoxy-benzylsulfanyl)-5-méthyl-hexènoate de méthyle

Rdt 33%. HPLC (80%): Tr = 8,3 et 9,3 min. RMN ¹H (CDCl₃) : 0,65 et 0,7 (d, 3H, CH-C**H**₃), 0,8 (d, 3H, CH-C**H₃**), 1,2-1,4 (m, 2H, C**H**₂-CH), 1,7 (m, 1H, C**H**-C**H**₃), 2,9 et 3,2 (m, 1H, -CH-S), 3,55 (s, 2H, -CH₂-S), 3,7 (s, 3H, CH₃O), 3,75 (s, 3H, CO₂CH₃), 4,6 et 4,65 (dd, 1H, CH-CO₂), 5,15 (s, 2H, O-C**H₂**-C₆H₅), 5,45 et 5,55 (d, 1H, NH), 6,75 (d, 2H, J = 7 Hz, S-C₆**H₄**), 7,05 et 7,1 (d, 2H, J = 7 Hz, S-C₆**H**₄), 7,43 (m, 5H, O-CH₂-C₆**H₅**).

### Composé 2d

### 2-Benzyloxycarbonylamino-3-(4-méthoxy-benzylsulfanyl)-4-phényl-butyrate de méthyle

Rdt 45%. HPLC (80%) : Tr = 8,0 et 8,4 min. RMN ¹H (CDCl₃) : 2,8, 2,90 et 3,05 (dd, 2H, S-CH-C**H₂**), 3,4 (m, 1H, S-C**H**-CH₂), 3,55 (s, 3H, CH₃O), 3,6 et 3,75 (s, 2H, -CH₂-S), 3,8 (s, 3H, CO₂CH₃), 4,55 (dd, 1H, CH-CO₂), 5,05 et 5,15 (s, 2H, O-C**H₂**-C₆H₅), 5,5 (d, 1H, NH), 6,75 (d, 2H, J = 7 Hz, S-C₆**H₄**), 7,05 (d, 2H, J = 7 Hz, S-C₆**H₄**), 7,1-7,43 (m, 10H, O-CH₂-C₆**H**₅ et CH₂-C₆**H**₅).

### Composé 2e

### 2-Benzyloxycarbonylamino-3-(4-méthoxy-benzylsulfanyl)-5-phényl-pentanoate de méthyle

Rdt 84%. HPLC (80%) : Tr = 8,3 et 9,1 min. RMN ¹H (CDCl₃) : 1,7-2,0 (m, 2H, CH-C**H₂**-CH₂), 2,5-2,75 (m, 2H, CH-CH₂-C**H₂**), 2,8 et 3,1 (m, 1H, S-C**H**-CH₂), 3,55 et 3,65 (s, 2H, -CH₂-S), 3,7 (s, 3H, CH₃O), 3,8 (s, 3H, CO₂CH₃), 4,6 et 4,7 (dd, 1H, CH-CO₂), 5,15 (s, 2H, O-C**H₂**-C₆H₅), 5,4 et 5,6 (d, 1H, NH), 6,80 (d, 2H, J = 7 Hz, S-C₆**H₄**), 7,0 et 7,1 (d, 2H, J = 7 Hz, S-C₆**H₄**), 7,2-7,43 (m, 10H, O-CH₂-C₆**H₅** et CH₂-C₆**H₅**).

### Composé 2f

### 2-Benzyloxycarbonylamino-3-(4-méthoxy-benzylsulfanyl)-5-(4-méthoxy-phényl)-pentanoate de méthyle

Rdt 71%. HPLC (80%) : Tr = 7,9 et 8,4 min. RMN ¹H (CDCl₃) : 1,7-1,9 (m, 2H, CH-C**H₂**-CH₂), 2,45 et 2,83 (m, 2H, CH-CH₂-C**H₂**), 2,86 et 3,1 (m, 1H, S-C**H**-CH₂), 3,55 et 3,65 (s, 2H, -CH₂-S), 3,7 (s, 3H, CH₃O), 3,8 (m, 6H, CO₂C**H**₃ et CH₃O), 4,65-4,7 (dd, 1H, CH-CO₂), 5,13 (s, 2H, O-C**H₂**-C₆H₅), 5,45 et 5,53 (d, 1H, NH), 6,8 (m, 4H, H Aromatique), 6,9-7,2 (m, 4H, H Aromatique), 7,3 - 7,4 (m, 5H, CH₂-C₆**H₅**).

### Composé 2g

### 2-Benzyloxycarbonylamino-3-(4-méthoxy-benzylsulfanyl)-5-(2-méthoxy-phényl)-pentanoate de méthyle

Rdt 62%. HPLC (80%) : Tr = 8,9 et 9,6 min. RMN ¹H (CDCl₃) : 1,7-1,9 (m, 2H, CH-C**H₂**-CH₂), 2,56 et 2,83 (m, 2H, CH-CH₂-C**H₂**), 2,86 et 3,1 (m, 1H, S-C**H**-CH₂), 3,55 et 3,7 (s, 2H, -CH₂-S), 3,63 et 3,66 (s, 3H, CH₃O), 3,8 (s, 6H, CO₂CH₃ et CH₃O), 4,66 et 4,76 (dd, 1H, CH-CO₂), 5,13 (s, 2H, O-C**H₂-**C₆H₅), 5,45 et 5,53 (d, 1H, NH), 6,76 -6,95 (m, 4H, H Aromatique), 7,03 (m, 1H, H Aromatique), 7,06 (d, 1H, H Aromatique), 7,15 - 7,25 (m, 2H, H Aromatique), 7,3 - 7,4 (m, 5H, CH₂-C₆**H₅**).

### Composé 2h

### 2-Benzyloxycarbonylamino-3-(4-méthoxy-benzylsulfanyl)-5-(4-bromo-phényl)-pentanoate de méthyle

Rdt 57%. HPLC (80%) : Tr = 15,1 et 15,9 min. RMN ¹H (CDCl₃) : 1,6-1,8 (m, 2H, CH-C**H₂**-CH₂), 2,5-2,7 (m, 2H, CH-CH₂-C**H₂**), 2,8 et 3,1 (m, 1H, S-C**H**-CH₂), 3,55 et 3,65 (s, 2H, -CH₂-S), 3,7 (s, 3H, CH₃O), 3,8 (m, 3H, CO₂CH₃), 4,6-4,7 (dd, 1H, CH-CO₂), 5,13 (s, 2H, O-C**H₂**-C₆H₅), 5,45 et 5,6 (d, 1H, NH), 6,6-6,8 (m, 4H, H Aromatique), 7,1-7,2 (m, 4H, H Aromatique), 7,3 - 7,4 (m, 5H, CH₂-C₆**H₅**).

### Préparation 3 : Synthèse des composés 3

A une solution de diéthoxyphosphorylméthanesulfonate de néopentyl (2 équivalents) dans du THF anhydre (4,5 ml/mmol) on ajoute goutte à goutte à -78°C, une solution de n-butyl lithium 1,6 M dans l'hexane (2 équivalents). Après 30 min sous agitation, on ajoute une solution du composé **2** (1 équivalent) dans du THF anhydre (1 ml/mmol) suivi de l'introduction goutte à goutte d'une solution d'hydrure de diisobutylaluminium 1,6 M dans le toluène (2 équivalents). Le mélange réactionnel est maintenu à -78°C 4 h, on laisse ensuite revenir à température ambiante une nuit. Les solvants sont éliminés sous pression réduite, le résidu est additionné de 10 ml/mmol d'éther et de 5 ml/mmol d'HCl 2N. Le mélange est agité 30 min, la phase organique est séparée, la phase aqueuse est extraite deux fois avec un volume d'éther équivalent. Les phases organiques sont réunies, séchées sur Na₂SO₄ et concentrées sous pression réduite. L'huile obtenue est purifiée par chromatographie sur silice. Après élimination des solvants le produit **3** est obtenu sous forme d'une huile.

On a obtenu de la même manière les composés 3a, 3b, 3c, 3d, 3e, 3f, 3g et 3h qui ont été caractérisés par leur spectre ¹H RMN dans CDCl₃ à 400 MHz.

### Composé 3a

### 3-Benzyloxycarbonylamino-4-(4-méthoxy-benzylsulfanyl)-pent-1-ène-1-sulfonate de 2,2-diméthylpropyle

Rdt 42%. HPLC (80%) : Tr = 8,1 et 8,4 min. RMN ¹H (CDCl₃) : 1,0 (s, 9H, C(CH₃)₃), 1,2 et 1,3 (d, 3H, J = 7 Hz, CH₃), 2,8 (m, 1H, -CH-S), 3,6-3,9 (m, 7H, SO₃CH₂, S-CH₂, CH₃O), 4,6 (m, 1H, CH-N), 5,15 (s, 2H, O-C**H₂**-C₆H₅), 5,2 (d, 1H, NH), 6,3 (d, 1H, CH=C**H**-SO₃), 6,7 (dd, 1H, C**H**=CH-SO₃), 6,8 (m, 2H, S-C₆**H₄**), 7,2 (m, 2H, S-C₆**H₄**), 7,43 (m, 5H, O-C**H**₂-C₆**H₅**).

### Composé 3b

### 3-Benzyloxycarbonylamino-4-(4-méthoxy-benzylsulfanyl)-4-phényl-but-1-ène-1-sulfonate de 2,2-diméthylpropyle

Rdt 42%. HPLC (80%) : Tr = 10,8 min. RMN ¹H (CDCl₃) : 0,9 (s, 9H, C(CH₃)₃), 3,4 (m, 2H, S-CH₂), 3,6 (s, 2H, SO₃CH₂), 3,8 (s, 3H, CH₃O), 3,9 (m, 1H, -CH-S), 4,8 (m, 1H, CH-N), 5,05 (s, 2H, O-CH₂-C₆H₅), 5,1 (d, 1H, NH), 6,1 (d, 1H, CH=C**H**-SO₃), 6,7 (dd, 1H, C**H**=CH-SO₃), 6,8 (m, 2H, S-C₆**H**₄), 7,2 (m, 2H, S-C₆**H₄**), 7,43 (m, 10H, H aromatique).

### Composé 3c

### 3-Benzyloxycarbonylamino-4-(4-méthoxy-benzylsulfanyl)-6-méthyl-hept-1-ène-1-sulfonate de 2,2-diméthylpropyle

Rdt 56%. HPLC (80%) : Tr = 14,4 et 15,4 min. RMN ¹H (CDCl₃) : 0,6 et 0,8 (d, 6H, J = 7 Hz, CH(CH₃)₂), 0,9 (s, 9H, C(CH₃)₃), 1,2-1,4 (m, 2H, C**H**₂-CH), 1,7 (m, 1H, C**H**(CH₃)₂), 2,7 (m, 1H, -CH-S), 3,6-3,9 (m, 7H, SO₃CH₂, S-CH₂, CH₃O), 4,6 (m, 1H, CH-N), 5,05 (s, 2H, O-C**H₂**-C₆H₅), 5,1 (d, 1H, NH), 6,3 (d, 1H, CH=C**H**-SO₃), 6,7 (dd, 1H, C**H**=CH-SO₃), 6,8 (m, 2H, S-C₆**H**₄), 7,2 (m, 2H, S-C₆**H₄**), 7,43 (m, 5H, H aromatique).

### Composé 3d

### 3-Benzyloxycarbonylamino-4-(4-méthoxy-benzylsulfanyl)-5-phényl-pent-1-ène-1-sulfonate de 2,2-diméthylpropyle

Rdt 76%. HPLC (80%) : Tr = 12,7 min. RMN ¹H (CDCl₃) : 0,9 (s, 9H, C(CH₃)₃), 2,8 (m, 3H, C**H**₂-C**H**-S), 3,6-3,9 (m, 7H, SO₃CH₂, S-CH₂, CH₃O), 4,6 (m, 1H, CH-N), 5,05 (s, 2H, O-C**H₂**-C₆H₅), 5,1 (d, 1H, NH), 6,3 (d, 1H, CH=C**H**-SO₃), 6,7 (dd, 1H, C**H**=CH-SO₃), 6,8 (m, 2H, S-C₆**H**₄), 7,0 (m, 2 H, H aromatique), 7,2 (m, 2H, S-C₆**H**₄), 7,43 (m, 8H, H aromatique).

### Composé 3e

### 3-Benzyloxycarbonylamino-4-(4-méthoxy-benzylsulfanyl)-6-phényl-hex-1-ène-1-sutfonate de 2,2-diméthylpropyle

Rdt 74%. HPLC (80%) : Tr = 15,1 et 16,0 min. RMN ¹H (CDCl₃) : 0,9 (s, 9H, C(CH₃)₃), 1,8 (m, 2H, C**H₂**-CH-S), 2,6 (m, 2H, C**H**₂-CH₂-CH-S), 2,8 (m, 1H, C**H**-S), 3,6-3,9 (m, 7H, SO₃CH₂, S-CH₂, CH₃O), 4,6-4,7 (m, 1H, CH-N), 5,05 et 5,2 (d, 1H, NH), 5,15 (s, 2H, O-C**H₂**-C₆H₅), 6,3 (d, 1H, CH=C**H**-SO₃), 6,7 (dd, 1H, C**H**=CH-SO₃), 6,8 (m, 2H, S-C₆**H₄**), 7,0-7,1 (m, 5 H, H aromatique), 7,2 (m, 2H, S-C₆**H₄**), 7,43 (m, 5H, H aromatique).

### Composé 3f

### 3-Benzyloxycarbonylamino-4-(4-méthoxy-benzylsulfanyl)-6-(4-méthoxy-phényl)-hex-1-ène-1-sulfonate de 2,2-diméthylpropyle

Rdt 59%. HPLC (80%) : Tr = 13,2 et 14,0 min. RMN ¹H (CDCl₃) : 0,9 (s, 9H, C(CH₃)₃), 1,8 (m, 2H, C**H**₂-CH-S), 2,6 (m, 2H, C**H**₂-CH₂-CH-S), 2,8 (m, 1H, C**H**-S), 3,6-3,9 (m, 10H, SO₃CH₂, S-CH₂, CH₃O), 4,6-4,7 (m, 1H, CH-N), 5,05 et 5,2 (d, 1H, NH), 5,15 (s, 2H, O-C**H₂**-C₆H₅), 6,3 (d, 1H, CH=C**H**-SO₃), 6,7 (dd, 1H, CH=CH-SO₃), 6,8 (m, 4H, H aromatique), 7,2 (m, 4H, H aromatique), 7,43 (m, 5H, H aromatique).

### Composé 3g

### 3-Benzytoxycarbonylamino-4-(4-méthoxy-benzylsulfanyl)-6-(2-méthoxy-phényl)-hex-1-ène-1-sulfonate de 2,2-diméthylpropyle

Rdt 62%. HPLC (80%B) : Tr = 15,74 et 16,17 min. ¹H RMN (CDCl₃) : 1 (s, 9H, C(CH₃)₃), 1,8 (m, 2H, CH₂-CH-S), 2,6 (m, 2H, C**H**₂-CH₂-CH-S), 2,8 (m, 1H, C**H**-S), 3,6 - 3,8 (m, 10H, SO₃CH₂, S-CH₂, CH₃O), 4,7-4,8 (m, 1H, CH-N), 5,15 (m, 2H, O-C**H**₂-C₆H₅), 5,2 (d, 1H, NH), 6,3 (d, 1H, CH=C**H-**SO₃), 6,7 (dd, 1H, CH=CH-SO₃), 6,8 (m, 2H, H aromatique), 7,03-7,25 (m, 4H, H aromatique), 7,43 (m, 7H, H aromatique).

### Composé 3h

### 3-Benzyloxycarbonylamino-4-(4-méthoxy-benzylsulfanyl)-6-(4-bromo-phényl)-hex-1-ène-1-sulfonate de 2,2-diméthylpropyle

Rdt 45%. HPLC (80%) : Tr = 21,8 et 23,3 min. RMN, ¹H (EDCl₃) : 0,9 (s, 9H, C(CH₃)₃), 1,8 (m, 2H, C**H**₂-CH-S), 2,6 (m, 2H, C**H**₂-CH₂-CH-S), 2,8 (m, 1H, C**H**-S), 3,6-3,9 (m, 7H, SO₃CH₂, S-CH₂, CH₃O), 4,6-4,7 (m, 1H, CH-N), 5,2 (d, 1H, NH), 5,15 (m, 2H, O-C**H₂**C₆H₅), 6,3 (d, 1H, CH=C**H**-SO₃), 6,7 (dd, 1H, C**H**=CH-SO₃), 6,8 (m, 2H, H aromatique), 6,9-7,2 (m, 4H, H aromatique), 7,43 (m, 7H, H aromatique).

### Préparation 4 : synthèse des composés 4

A une solution d'un composé **3** (1 équivalent) dans de l'éthanol absolu (5 ml/mmol) on ajoute du borohydrure de sodium (1 équivalent). Le mélange réactionnel est agité à température ambiante une nuit. Les solvants sont éliminés sous pression réduite, le résidu est additionné de 10 ml/mmol d'acétate d'éthyle et de 5 ml/mmol d'eau. La phase organique est séparée, la phase aqueuse est extraite deux fois avec un volume d'acétate d'éthyle équivalent. Les phases organiques sont réunies, séchées sur Na₂SO₄ et concentrées sous pression réduite. L'huile obtenue est purifiée par HPLC semi-préparative. Après élimination des solvants le produit **16** est obtenu sous forme d'une huile.

On a obtenu de la même manière les composés 4a, 4b, 4c, 4d, 4e, 4f, 4g et 4h qui ont été caractérisés par leur spectre ¹H RMN dans CDCl₃ à 400 MHz et par spectroscopie de masse electrospray.

### Composé 4a

### 3-Benzyloxycarbonylamino-4-(4-méthoxy-benzylsulfanyl)-pentane-1-sulfonate de 2,2-diméthylpropyle

Rdt 30%. HPLC (80%) : Tr = 7,5 et 7,7 min. RMN ¹H (CDCl₃) : 1,0 (s, 9H, C(CH₃)₃), 1,2 et 1,3 (d, 3H, J = 7 Hz, CH₃), 1,8-2,1 (m, 2H, C**H**₂CH₂-SO₃), 2,8 (m, 1H, -CH-S), 3,0 (m, 2H, CH₂-C**H**₂-SO₃), 3,6-3,9 (m, 8H, SO₃CH₂, C**H**-S-C**H**₂, CH₃O), 4,7-5,0 (d, 1H, NH), 5,15 (m, 2H, O-C**H₂**-C₆H₅), 6,8 (m, 2H, S-C₆**H₄**), 7,2 (m, 2H, S-C₆**H₄**), 7,43 (m, 5H, O-CH₂-C₆**H₅**). ES⁺ : 546 M+Na⁺

### Composé 4b

### 3-Benzyloxycarbonylamino-4-(4-méthoxy-bonzylsulfanyl)-4-phényl-butane-1-sulfonate de 2,2-diméthylpropyle

Rdt 62%. HPLC (80%): Tr = 10,3 min. RMN ¹H (EDCl₃) : 1,0 (s, 9H, C(CH₃)₃), 1.8 (m, 1H, CH₂CH₂-SO₃), 2,1 (m, 1H, CH₂CH₂-SO₃), 3,2 (m, 2H, CH₂-CH₂-SO₃), 3,4 (m, 1H, -CH-S), 3,7-3,8 (m, 7H, SO₃CH₂, S-CH₂, CH₃O), 4,05 (m, 1H, CH-S), 4,8 (d, 1H, NH), 5,15 (m, 2H, O-CH₂-C₆H₅), 6,8 (m, 2H, S-C₆H₄). 7,05 (m, 2H, S-C₆H₄), 7,43 (m, 10H,H aromatique). ES⁺ : 608 M+Na⁺

### Composé 4c

### 3-Benzyloxycarbonylamino-4-(4-méthoxy-benzylsulfanyi)-6-méthyl-heptane-1-sulfonate de 2,2-diméthylpropyle

Rdt 80%. HPLC (80%) : Tr = 13,8 et 14,5 min. RMN ¹H (CDCl₃) : 0,6 et 0,8 (d, 6H, J = 7 Hz, CH(CH₃)₂), 0,9 (s, 9H, C(CH₃)₃), 1,2-1,4 (m, 2H, CH₂-CH), 1,6-1,8 (m, 2H, CH₂CH₂-SO₃), 2,0 (m, 1H, CH(CH₃)₂), 2,7 (m, 1H, - CH-S), 3,2 (m, 2H, CH₂-CH₂-SO₃), 3,6-3,9 (m, 8H, SO₃CH₂, S-CH₂, CH₃O, CH-N), 4,9 (d, 1H, NH), 5,05 (s, 2H, O-CH₂C₆H₅), 6,8 (m, 2H, S-C₆H₄), 7,2 (m, 2H, S-C₆H₄), 7,43 (m, 5H, H aromatique). ES⁺ : 588 M+Na⁺

### Composé 4d

### 3-Benzyloxycarbonylamino-4-(4-méthoxy-benzylsulfanyl)-5-phényl-pentane-1-sulfonate de 2,2-diméthylpropyle

Rdt 76%. HPLC (80%) : Tr = 12,17 min. RMN ¹H (CDCl₃) : 0,9 (s, 9H, C(CH₃)₃), 1,6-1,8 (m, 2H, CH₂CH₂-SO₃), 2,6-3,1 (m, 5H, CH₂-CH-S, CH₂CH₂-SO₃), 3,4-3,6 (m, 2H, S-CH₂), 3,7-3,9 (m, 6H, SO₃CH₂, CH-N, CH₃O) 4,9 (d, 1H, NH), 5,15 (s, 2H, O-CH₂-C₆H₅), 6,75 (m, 2H, S-C₆H₄), 7,1 (m, 3 H, H aromatique), 7,2 (m, 2H, H aromatique), 7,3-7,4 (m, 7H, H aromatique). ES⁺ : 622 M+Na⁺

### Composé 4e

### 3-Benzyloxycarbonylamino-4-(4-méthoxy-benzylsulfanyl)-6-phényl-hexane-1-sulfonate de 2,2-diméthylpropyle

Rdt 74%. HPLC (90%) : Tr = 15,6 et 16,5 min RMN ¹H (CDCl₃) : 0,9 (s, 9H, C(CH₃)₃), 1,8-2,1 (m, 4H, CH₂-CH-S, CH₂-CH₂-SO₃), 2,5 (m, 2H CH₂-CH₂), 2,7-3,1 (m, 3H, CH-S, CR₂CH₂-SO₃), 3,6-3,8 (m, 7H, SO₃CH₂, S-CH₂, CH₃O), 3,95 (m, 1H, CH-N), 4,85 et 4,95 (d, 1H, NH), 5,15 (s, 2H, O-CH₂-C₆H₅), 6,8 (m, 2H, S-C₆H₄), 7,0-7,2 (m, 5 H, H aromatique), 7,2 (m, 2H, S-C₆H₄), 7,43 (m, 5H, H aromatique). ES⁺ : 636 M+Na⁺

### Composé 4f

### 3-Benzyloxycarbonylamino-4-(4-méthoxy-benzylsulfanyl)-6-(4-méthoxy-phényl)-hexane-1-sulfonate de 2,2-diméthylpropyle

Rdt 59%. HPLC (80%) : Tr = 12,6 et 13,4 min. RMN ¹H (CDCl₃) : 0,9 (s, 9H, C(CH₃)₃), 1,8-2,1 (m, 4H, CH₂-CH-S, CH₂-CH₂-SO₃), 2,5 (m, 2H, CH₂-CH₂), 2,7-3,1 (m, 3H, CH-S, CH₂CH₂-SO₃), 3,6-3,8 (m, 10H, SO₃CH₂, S-CH₂, CH₃O, CH₃O), 3,95 (m, 1H, CH-N), 4,85 et 4,95 (d, 1H, NH), 5,15 (s, 2H, O-CH₂-C₆H₅), 6,8 (m, 4H, H aromatique), 6,9-7,1 (m, 4 H, H aromatique), 7,43 (m, 5H, H aromatique). ES⁺ : 666 M+Na⁺

### Composé 4g

### 3-Benzyloxycarbonylamino-4-(4-méthoxy-benzylsulfanyl)-6-(2-méthoxy-phényl)-hexane-1-sulfonate de 2,2-diméthylpropyle

Rdt 83%. HPLC (80%) : Tr = 14,7 et 15,3 min, RMN ¹H (CDCl₃) : 0,9 (s, 9H, C(CH₃)₃), 1,7-2,1 (m, 4H, CH₂-CH-S, CH₂-CH₂-SO₃), 2,4 (m, 2H, CH₂-CH₂), 2,7-3,1 (m, 3H, CH-S, CH₂CH₂SO₃), 3,6-3,8 (m, 10H, SO₃CH₂, S-CH₂, CH₃O, CH₃O), 4,0 (m, 1H, CH-N), 4,85 et 4,95 (d, 1H, NH), 5,15 (m, 2H, O-CH₂-C₆H₅), 6,8 (m, 4H, H aromatique), 6,9-7,1 (m, 4 H, H aromatique), 7,43 (m, 5H, H aromatique). ES⁺ : 666 M+Na⁺

### Composé 4h

### 3-Benzyloxycarbonylamino-4-(4-méthoxy-benzylsulfanyl)-6-(4-bromo-phényl)-hexane-1-sulfonate de 2,2-diméthylpropyle

Rdt 91%. HPLC (80%) : Tr = 21,7 et 22,8 min. RMN ¹H (CDCl₃) : 0,9 (s, 9H, C(CH₃)₃), 1,6-1,9 (m, 4H, CH₂-CH-S, CH₂-CH₂-SO₃), 2,45 (m, 2H, CH₂-CH₂), 2,65 (m, 1H, CH-S), 3,1 (m, 2H, CH₂CH₂-SO₃), 3,5-3,8 (m, 7H, SO₃CH₂, S-CH₂, CH₃O), 3,95 (m, 1H, CH-N), 4,85 et 4,95 (d, 1H, NH), 5,1 (2H, s, O-CH₂-C₆H₅), 6,8 (m, 4H, H aromatique), 6,9-7,1 (m, 4 H, H aromatique), 7,43 (m, 5H, H aromatique). ES⁺ : 700-702 M+Na⁺

### Préparation 5 : synthèse des composés 5

Au composé **4** (1 équivalent) on ajoute de l'anisole (5 équivalents) et de l'acide trifluoro acétique (7 ml/mmol). Le mélange réactionnel est porté à reflux sous argon 16 h. Le solvant est éliminé sous pression réduite. Le résidu est suspendu dans 5 ml/mmol de cyclohexane qui est ensuite éliminé sous pression réduite. Cette opération est répétée deux fois afin d'éliminer les traces d'acide trifluoroacétique. L'huile obtenue est additionnée d'éther, l'inhibiteur **5** précipite et est séché sous pression réduite après filtration.

On a obtenu de la même manière les composés 5a, 5b, 5c, 5d, 5e, 5f, 5g et 5h qui ont été caractérisés par leur spectre ¹H RMN dans CDCl₃ à 400 MHz et par spectroscopie de masse electrospray.

### Composé 5a

### Acide 3-amino-4-mercapto-pentane-1-sulfonique

Rdt 75%. RMN ¹H (DMSO-D₆) : 1,2 (d, 3H, J = 7 Hz, CH₃), 1,8-2,0 (m, 2H, CH₂CH₂-SO₃), 2,6 (m, 2H, CH₂-CH₂-SO₃), 2,8 (m, 1H, -CH-S), 3,1 (m, 1H, CHN), 7,9 (s, 3H, NH₃⁺). ES⁺ : 222 M+Na⁺. ES⁻ : 198 M-H

### Composé 5b

### Acide 3-amino-4-mercapto-4-phényl-butane-1-sulfonique

Rdt 69%. RMN ¹H (DMSO-D₆) : 1,8-2,1 (m, 2H, CH₂CH₂-SO₃), 2,5-2,7 (m, 3H, CH₂-CH₂-SO₃; CHS), 3,6 (m, 1H, CHN), 7,1 (m, 5 H,H aromatique), 7,9 (s, 3H, NH₃⁺), ES⁺ : 284 M+Na⁺, ES⁻ : 260 M-H

### Composé 5c

### Acide 3-amino-4-mercapto-6-méthyl-heptane-1-sulfonique

Rdt 50%. RMN ¹H (DMSO-D₆) : 0,7 et 0,8 (d, 6H, J = 7 Hz, CH(CH₃)₂), 1,35 (m, 2H, CH₂-CH(CH₃)₂), 1,7-1,9 (m, 2H, CH₂CH₂-SO₃ ), 2,0 (m, 1H, CH(CH₃)₂), 2,7 (m, 2H, CH₂CH₂-SO₃), 3,0 (m, 1H, CHS), 3,3 (m, 1H, CH-N), 7,9 (s, 3H, NH₃⁺). ES⁻ : 240 M-H

### Composé 5d

### Acide 3-amino-4-mercapto-5-phényl-pentane-1-sulfonique

Rdt 82%. RMN ¹H (DMSO-D₆) : 1,9-2,1 (m, 2H, CH₂CH₂-SO₃), 2,6-2,7 (m, 4H, CH₂-CH-S, CH₂CH₂-SO₃), 2,9-3,1 (m, 1H, CHS), 3,4 (m, 1H, CH-N), 7,1 (m, 5 H, H aromatique), 7,9 (s, 3H, NH₃⁺). ES⁺ : 298 M+Na⁺. ES⁻ : 274 M-H

### Composé 5e

### Acide 3-amino-4-mercapto-6-phényl-hexane-1-sulfonique

Rdt 55%. RMN ¹H (DMSO-D₆) : 1,6 (m, 1H, CH₂-CH-S), 1,7-2,0 (m, 3H, CH₂-CH₂-CH-S, CH₂CH₂-SO₃), 2,6-2,7 (m, 3H, CH₂-CH-S, CH₂CH₂-SO₃), 2,8 (m, 1H, CH₂-CH-S), 2,9 (m, 1H, CHS), 3,4 (m, 1H, CH-N), 7,1 (m, 5 H, H aromatique), 7,9 (s, 3H, NH₃⁺), ES⁻ : 288 M-H

### Composé 5f

### Acide 3-amino-4-mercapto-6-(4-méthoxy-phényl)-hexane-1-sulfonique

Rdt 51%. RMN ¹H (DMSO-D₆) : 1,6 (m, 1H, CH₂-CH-S), 1,7-2,0 (m, 3H, CH₂-CH₂-CH-S, CH₂CH₂-SO₃), 2,6-2,8 (m, 4H, CH₂-CH-S, CH₂CH₂-SO₃), 2,9 (m, 1H, CHS), 3,4 (m, 1H, CH-N), 3,5-3,8 (m, 3H, OCH₃), 6,7 (m, 2 H, H aromatique), 7,0 (m, 2H, H aromatique), 7,9 (s, 3H, NH₃⁺). ES⁺ : 342 M+Na⁺.

### Composé 5g

### Acide 3-amino-4-mercapto-6-(2-méthoxy-phényl)-hexane-1-sulfonique

Rdt 80%. RMN ¹H (DMSO-D₆) : 1,3-1,6 (m, 2H, CH₂-CHS), 1,7-2,1 (m, 2H, CH₂CH₂-SO₃), 2,5-2,8 (m, 4H, CH₂-CH-S, CH₂CH₂-SO₃), 2,9 (m, 1H, CHS), 3,4 (m, 1H, CH-N), 3,5-3,8 (m, 3H, OCH₃), 6,6-7,3 (m, 4 H, H aromatique), 7,8 (s, 3H, NH₃⁺). ES⁺ : 342 M+Na⁺.

### Composé 5h

### Acide 3-amino-4-mercapto-6-(4-bromo-phényl)-hexane-1-sulfonique

Rdt 85%. RMN ¹H (DMSO-D₆) : 1,6 (m, 1H, CH₂-CH-S), 1,8-2,0 (m, 3H, CH₂-CH₂-CHS, CH₂CH₂-SO₃), 2,6 (m, 3H, CH₂-CH-S, CH₂CH₂-SO₃). 2,8 (m, 1H, CH₂-CH-S), 2,9 (m, 1H, CHS), 3,4-3,5 (m, 1H, CH-N), 7,1 (m, 2 H, H aromatique). 7,4 (m, 2H, H aromatique), 7,9 (s, 3H, NH₃⁺). ES⁺ : 390-392 M+Na⁺.

### Préparation 6 : synthèse du composé 6

### (2E)-5-phényl-pent-2-ènoate d'éthyle

A 32,6 ml (164 mmol, 1,1 équivalent) de triéthylphosphonoacétate dans 150 ml de dichlorométhane anhydre, on ajoute sous argon à 0°C goutte à goutte 24,7 ml (164 mmol, 1,1 équivalent) de diazabicycloundécène (DBU). Le mélange est agité 30 min. Après retour à 25°C, on additionne 19,6 ml (149 mmol, 1 équivalent.) d'hydrocinnamaldéhyde. Après 16 h de réaction on additionne au milieu réactionnel 150 ml HCl 1N. La phase organique est séparée, lavée avec 2 x 50 ml HCl 1N, puis 2 x 50 ml NaHCO₃ 10%. La phase organique est séchée sur Na₂SO₄, concentrée sous pression réduite. Le produit est purifié par filtration sur silice. On obtient une huile incolore.

Rdt 67%. RMN ¹H (CDCl₃) : 1,3 (t, 3H, J = 7 Hz, CH₃-CH₂), 2,5 (q, 2H, J = 7 Hz, Ph-CH₂-CH₂), 2,8 (t, 2H, J = 7 Hz, Ph-CH₂-CH₂), 4,2 (q, 2H, J = 7 Hz, CH₃-CH₂), 5,85 (d, 1H, J = 12 Hz, CH=CH-CO), 7,0 (td, 1H. J = 7 Hz. J = 12 Hz, CH=CH-CO), 7,2 (m, 3 H, H aromatique). 7,3 (m, 2H, H aromatique).

### Préparation 7 : synthèse des composés 7 a-b

A une solution de 50g (1,4 g/mmol) d'AD-mix et de 0,36g (3,7 mmol, 0,01 g/mmol) du composé 6 dans un mélange de 160 ml (4,5 ml/mmol) d'alcool tertiobutylique et de 160 ml (4,5ml/mmol) d'eau, on ajoute à 25°C 7,3g (35,7 mmol, 1 équivalent) de (2Z)-5-phényl-pent-2-ènoate d'éthyle. Après 5 h de réaction, on additionne 45 g (357 mmol, 1,3 g/mmol, 10 équivalents) de sulfite de sodium, le mélange est agité 30 min. Après dilution du milieu réactionnel par 150 ml d'acétate d'éthyle, la phase organique est séparée, séchée sur Na₂SO₄ puis concentrée sous pression réduite. Le produit est purifié par filtration sur silice. On obtient une huile incolore.

Les composés 7a et 7b ont été caractérisés par chromatographie sur couche mince (CCM) sur un support de silice avec un éluant cyclohexane : acétate d'éthyle, par leur spectre ¹H RMN dans CDCl₃ à 400 MHz et par leur pouvoir rotatoire spécifique.

### Composé 7a

### (2R,3S)-2,3-dihydroxy-5-phényl-pentanoate d'éthyle

Rdt: 70%. CCM (cyclohexane : acétate d'éthyle, 60 : 40) : R*_{f}*=0,28. [α]20D = = -29,8° (c = 1, MeOH). HPLC (50% B) : Rt = 5,5 min. RMN ¹H (CDCl₃) : 1,3 (t, 3H, COOCH₂CH₃), 1,95 (m, 3H, OH, CH₂C-OH), 2,74 (m, 1H, CH₂Phe), 2,85 (m, 1H, CH₂Phe), 3,08 (d, 1H, OH), 3,93 (m, 1H, CHCH₂), 4,2 (d, 1H, CHCOOEt), 4,3 (q, 2H, COOCH₂CH₃), 7,15 - 7,35 (m, 5H, Ph).

### Composé 7b

### (2S,3R)-2,3-dihydroxy-5-phényl-pentanoate d'éthyle

Rdt : 72%. CCM (cyclohexane : acétate d'éthyle, 70 : 30) : R*_{f}*= 0,17. [α]20D = = +28,7° (c = 1,1, MeOH). HPLC (70%B) : Rt = 3,6 min. ¹H NMR (CDCl₃) : 1,3 (t, 3H, COOCH₂CH₃), 1,95 (m, 2H, CH₂ C-OH), 2,75 (m, 1H, CH₂Phe), 2,85 (m, 1H, CH₂Phe), 3,93 (m, 1H, CHCH₂), 4,1 (d, 1H, CHCOOEt), 4,3 (q, 2H, COOCH₂CH₃), 7,15 - 7,35 (m, 5H, Ph).

### Préparation 8 : synthèse des sulfates cycliques 8 a-b

A une solution de 6,9 g (29 mmol, 1 équivalent) de composé **7** et de 8 ml (58 mmol, 2 équivalents) de triéthylamine dans 60 ml (2 ml/ mmol) de dichlorométhane anhydre on additionne à 0°C au goutte à goutte 2,1 ml (29 mmol, 1 équivalent) de chlorure de thionyle en 10 min. Le mélange est agité 5 min, dilué par 40 ml d'éther et lavé avec 30 ml d'eau. Le solvant est éliminé sous pression réduite et le résidu est suspendu dans un mélange de 180 ml d'eau, 80 ml de chloroforme et de 80 ml de tétrachlorure de carbone puis additionné de 9,6 g (45 mmol 1,5 équivalents) de périodate de sodium et d'une quantité catalytique de RuCl₃. Après 1 h d'agitation à 25°C. le mélange est dilué par 150 ml d'éther, filtré sur Cellite^{®}. La phase organique est lavée avec 40 ml NaHCO₃ 10%, séchée sur Na₂SO₄ puis concentrée sous pression réduite. Le produit est purifié par filtration sur silice. On obtient une huile incolore.

Les composés 8a et 8b ont été caractérisés par CCM sur un support de silice avec un éluant cyclohexane : acétate d'éthyle, par leur spectre ¹H RMN dans CDCl₃ à 400 MHz et par leur pouvoir rotatoire spécifique.

### Composé 8a :

Rdt : 78%. TLC (cyclohexane : acétate d'éthyle, 60 : 40) : R*_{f}*=0,4. [α]20D = = -75,66° (c = 1, MeOH). HPLC (70%B) : Rt = 7,2 min RMN ¹H (CDCl₃) : 1,3 (t, 3H, COOCH₂CH₃), 2,3 (m, 2H, CH₂ C-OH), 2,76 (m, 1H, CH₂Phe), 2,89 (m, 1H, CH₂Phe), 4,3 (q, 2H, COOCH₂CH₃), 4,87 (d, 1H, CHCOOEt), 4,9 (m, 1H, CHCH₂), 7,2 - 7,4 (m, 5H, Ph).

### Composé 8b :

Rdt : 70%. CCM (cyclohexane: acétate d'éthyle, 60 : 40) : R*_{f}*=0,4. [α]20D = = +78,3° (c = 1,1, MeOH). HPLC (70%B) : Rt = 7,24 min. ¹H NMR (CDCl₃) : 1,3 (t, 3H, COOCH₂CH₃), 2,3 (m, 2H, CH₂ C-OH), 2,8 (m, 1H, CH₂Phe), 2,95 (m, 1H, CH₂Phe), 4,3 (q, 2H, COOCH₂CH₃), 4,87 (d, 1H, CHCOOEt), 4,9 (m, 1H, CHCH₂), 7,15 - 7,4 (m, 5H, Ph).

### Préparation 9 : synthèse des composés 9 a-b

A une solution de 3,3 g (11 mmol, 1 équivalent) du composé **8** dans 110 ml (10 ml/mmol) de THF anhydre, on ajoute 3,8 g (44 mmol, 4 équivalents) de bromure de lithium. Le mélange est agité à 25°C jusqu'à complète disparition du composé **8**. Après concentration sous pression réduite, le résidu est repris par 150 ml d'éther et 20 ml d'eau, puis additionné de 0,1 ml H₂SO₄ 20%. La solution est agitée 24 h à 4°C. La phase organique est séparée, lavée avec 3 x 20 ml NaHCO₃ 10%, séchée sur Na₂SO₄ puis concentrée sous pression réduite. Le produit est purifié par filtration sur silice. On obtient une huile incolore.

Les composés 9a et 9b ont été caractérisés par CCM sur un support de silice avec un éluant cyclohexane : acétate d'éthyle, par leur spectre ¹H RMN dans CDCl₃ à 400 MHz et par leur pouvoir rotatoire spécifique.

### Composé 9a

### (2S,3S)-2-bromo-3-hydroxy-5-phényl-pentanoate d'éthyle

Rdt : 85%. CCM (cyclohexane : acétate d'éthyle, 60 : 40) : R*_{f}*=0,5. [α]²⁰_{D} = -37,35° (c = 1,004, MeOH). HPLC (70%B) : Rt = 6,6 min . RMN ¹H (CDCl₃) 1,3 (t, 3H, COOCH₂CH₃), 1,83 (m, 1H, CH₂ C-OH), 2,17 (m, 1H, CH₂ C-OH), 2,75 (m, 2H, OH, CH₂Phe), 2,9 (m, 1H, CH₂Phe), 4,03 (m, 1H, CHCH₂), 4,15 (d, 1H, CHCOOEt), 4,27 (q, 2H, COOCH₂CH₃), 7,2-7,35 (m, 5H, Ph).

### Composé 9b

### (2R,3R)-2-bromo-3-hydroxy-5-phényl-pentanoate d'éthyle

Rdt : 91%. CCM (cyclohexane: acétate d'éthyle, 60 : 40) : R*_{f}*=0,5. [α]²⁰_{D} = +35,8° (c = 1,85, MeOH). HPLC (70%B) : Rt = 6,6 min RMN ¹H (CDCl₃) : 1,3 (t, 3H, COOCH₂CH₃), 1,83 (m, 1H, CH₂ C-OH), 2,17 (m, 1H, CH₂ C-OH), 2,75 (m, 2H, OH, CH₂Phe), 2,9 (m, 1H, CH₂Phe), 4,03 (m, 1H, CHCH₂), 4,15 (d, 1H, CHCOOEt), 4,27 (q, 2H, COOCH₂CH₃), 7,2-7,35 (m, 5H, Ph).

### Préparation 10 : synthèse des composés 10a-d

1) A une solution de 1,2 g (4 mmol, 1 équivalent) du composé **8** dans 24 ml (6 ml/mmol) d'acétone et 2,5 ml (0,6 ml/mmol) d'eau, on ajoute 0,32 g (5 mmol, 1,25 équivalent) d'azoture de sodium. Le mélange est agité à 25°C jusqu'à complète disparition du composé **8**. Après concentration sous pression réduite, le résidu est repris par 50 ml d'éther et 5 ml d'eau, puis additionné de 1 ml H₂SO₄ 20%. La solution est agitée 24 h à 4°C. La phase organique est séparée, lavée avec 3 x 20 ml NaHCO₃ 10%, séchée sur Na₂SO₄ puis concentrée sous pression réduite. Le produit est purifié par filtration sur silice. On obtient une huile incolore.
   Les composés 10a, 10b, 10c et 10d ont été caractérisés par CCM sur un support de silice avec un éluant cyclohexane : acétate d'éthyle, par leur spectre ¹H RMN dans CDCl₃ à 400 MHz et par leur pouvoir rotatoire spécifique.

### Composé 10a

### (2S,3S)-2-Azido-3-hydroxy-5-phényl-pentanoate d'éthyle

Rdt : 84%. CCM (cyclohexane : acétate d'éthyle, 60 : 40) : R*_{f}*=0,55. [α]²⁰_{D} = -28,7° (c = 1, MeOH). HPLC (70%B): Rt = 5,65 min. RMN ¹H (CDCl₃) : 1,3 (3H, t, COOCH₂CH₃), 1,9 (2H, m, CH₂ C-OH), 2,37 (1H, d, OH), 2,7 (1H, m, CH₂Phe), 2,9 (1H, m, CH₂Phe), 3,95 (1H, m, CHCH₂), 3,97 (1H, d, CHCOOEt), 4,3 (2H, q, COOCH₂CH₃), 7,2 - 7,35 (5H, m, Ph).

### Composé 10c

### (2R,3S)-2-Azido-3-hydroxy-5-phényl-pentanoate d'éthyle

Rdt : 86%. CCM (cyclohexane : acétate d'éthyle, 60 : 40) : R*_{f}*= 0,58. [α]²⁰_{D} = +15° (c = 1,082, MeOH). HPLC (70%B) : Rt = 5,72 min RMN ¹H (CDCl₃) : 1,35 (t, 3H, COOCH₂CH₃), 1,86 (m, 1H, CH₂ C-OH), 1,96 (m, 1H, CH₂ C-OH), 2,15 (d, 1H, OH), 2,70 (m, 1H, CH₂Phe), 2,85 (m, 1H, CH₂Phe), 3,9 (d, 1H, CHCOOEt), 4,07 (m, 1H, CHCH₂), 4,3 (q, 2H, COOCH₂CH₃), 7,15-7,35 (m, 5H, Ph).
2) A 2,6 g (8,6 mmol, 1 équivalent) du composé **9** dans 9 ml (1 ml/mmol) de diméthylsulfoxyde anhydre, on additionne 1,12 g (17,2 mmol, 2 équivalents) d'azoture de sodium. Le milieu réactionnel est agité à 25°C une nuit, puis dilué par 90 ml d'un mélange de cyclohexane et dichlorométhane 2:1. La phase organique est lavée avec 3 x 10 ml H₂O, 1 x 10 ml d'une solution saturée de NaCl et séchée sur Na₂SO₄ puis concentrée sous pression réduite. Le produit est purifié par filtration sur silice. On obtient une huile incolore.

### Composé 10b

### (2R,3R)-2-Azido-3-hydroxy-5-phényl-pentanoate d'éthyle

Rdt : 84%. CCM (cyclohexane : acétate d'éthyle, 70 : 30) : R*_{f}*=0,38. [α]²⁰_{D} = +28,5° (c = 1, MeOH). HPLC (70%B) : Rt = 5,63 min RMNi ¹H (CDCl₃) : 1,3 (3H, t, COOCH₂CH₃), 1,9 (2H, m, CH₂ C-OH), 2,37 (1H, d, OH), 2,7 (1H, m, CH₂Phe), 2,9 (1H, m, CH₂Phe), 3,95 (1H, m, CHCH₂), 3,97 (1H, d, CHCOOEt), 4,3 (2H, q, COOCH₂CH₃), 7,2 - 7,35 (5H, m, Ph).

### Composé 10d

### (2S,3R)-2-Azido-3-hydroxy-5-phényl-pentanoate d'éthyle

Rdt : 69%. CCM (cyclohexane : acétate d'éthyle, 60 : 40) : R*_{f}*=0,58. [α]²⁰_{D} = -11,7° (c = 0,95, MeOH). HPLC (70%B) : Rt = 5,76 min RMN ¹H (CDCl₃) 1,35 (t, 3H, COOCH₂CH₃), 1,86 (m, 1H, CH₂ C-OH), 1,96 (m, 1H, CH₂ C-OH), 2,15 (d, 1H, OH), 2,70 (m, 1H, CH₂Phe), 2,85 (m, 1H, CH₂Phe), 3,9 (d, 1H, CHCOOEt), 4,07 (m, 1H, CHCH₂), 4,3 (q, 2H, COOCH₂CH₃), 7,15-7,35 (m, 5H, Ph).

### Préparation 11 : synthèse des composés 11a-d

A 1,5 g (5,7 mmol, 1 équivalent) du composé **10**, dans 23 ml (4 ml/mmol) d'acétonitrile, on ajoute 1,49 g (5,7 mmol, 1 équivalent) de triphénylphosphine. Le mélange est agité une heure à 25°C puis porté à reflux cinq heures. Après concentration du milieu réactionnel sous pression réduite, l'huile est purifiée par filtration sur silice. On obtient une huile incolore.

Les composés 11a, 11b, 11c et 11d ont été caractérisés par CCM sur un support de silice avec un éluant cyclohexane : acétate d'éthyle, par leur spectre ¹H RMN dans CDCl₃ à 400 MHz et par leur pouvoir rotatoire spécifique.

### Composé 11a

### (2S,3R)-3-Phénéthyl-aziridine-2-carboxylate d'éthyle

Rdt: 84%. CCM (cyclohexane: acétate d'éthyle, 60: 40) : R*_{f}*= 0,26. [α]²⁰_{D} = +70° (c = 1,024, MeOH). HPLC (40%B) : Rt = 4,84 min . RMN ¹H (CDCl₃) : 1,3 (t, 3H, COOCH₂CH₃), 1,8 (m, 2H, CH₂ C-NH), 2,3 (m, 1H, CHCH₂), 2,33 (s, 1H, CHCOOEt), 2,8 (m, 2H, CH₂Phe), 4,2 (q, 2H, COOCH₂CH₃), 7,2 - 7,35 (m, 5H, Ph).

### Composé 11b

### (2R,3R)-3-Phénéthyl-aziridine-2-carboxylate d'éthyle

Rdt: 68%. CCM (cyclohexane : acétate d'éthyle, 50 : 50) : R*_{f}*=0,31. [α]²⁰_{D} = -14,38° (c = 1,05, MeOH). HPLC (40%B) : Rt = 4,15 min . RMN ¹H (CDCl₃) : 1,3 (t, 3H, COOCH₂CH₃), 1,85 (m, 1H, CH₂ C-NH), 1,97 (m, 1H, CH₂ C-NH), 2,27 (m, 1H, CHCH₂), 2,63 (d, 1H, CHCOOEt), 2,68 - 2,85 (m, 2H, CH₂Phe), 4,18 (2q, 2H, COOCH₂CH₃), 7,1-7,25 (m, 3H, Ph), 7,25-7,35 (m, 2H, Ph).

### Composé 11c

### (2R,3S)-3-Phénéthyl-aziridine-2-carboxylate d'éthyle :

Rdt : 72%. CCM (cyclohexane : acétate d'éthyle, 70 : 30) : R*_{f}*=0,2. [α]²⁰_{D} = -67,38° (c = 1, MeOH). HPLC (70%B) : Rt = 2,73 min . RMN ¹H (CDCl₃) : 1,3 (t, 3H, COOCH₂CH₃), 1,8 (m, 2H, CH₂ C-NH), 2,3 (m, 1H, CHCH₂), 2,33 (s, 1H, CHCOOEt), 2,8 (m, 2H, CH₂Phe), 4,2 (q, 2H, COOCH₂CH₃), 7,2 - 7,35 (m, 5H, Ph).

### Composé 11d

### (2S,3S)-3-Phénéthyl-aziridine-2-carboxylate d'éthyle

Rdt: 50%. CCM (cyclohexane: acétate d'éthyle, 50: 50): R*_{f}*=0,31. [α]²⁰_{D} = +12,83 (c = 1, MeOH). HPLC (40%B) : Rt = 4,07 min. RMN ¹H (CDCl₃) : 1,3 (t, 3H, COOCH₂CH₃), 1,85 (m, 1H, CH₂ C-NH), 1,97 (m, 1H, CH₂ C-NH), 2,27 (m, 1H. CHCH₂), 2,63 (d, 1H, CHCOOEt), 2,68 - 2,85 (m, 2H, CH₂Phe), 4,18 (2q, 2H, COOCH₂CH₃), 7,1-7,25 (m, 3H, Ph), 7,25-7,35 (m, 2H, Ph).

### Préparation 12 : synthèse des composés 12 a-d

A 0,5 g (2,3 mmol, 1 équivalent) du composé **11**, dans 9 ml (4 ml/mmol) de pyridine, on ajoute 1,14 g (4,6 mmol, 2 équivalent) de benzyloxycarbonylsuccinimide et 0,03 g (0,24 mmol, 0,1 équivalent) de 4-diméthylaminopyridine. Le mélange est agité 24 h à 4°C. Après concentration du milieu réactionnel sous pression réduite, l'huile est purifiée par filtration sur silice. On obtient une huile incolore.

Les composés 12a, 12b, 12c et 12d ont été caractérisés par CCM sur un support de silice avec un éluant cyclohexane : acétate d'éthyle, par leur spectre ¹H RMN dans CDCl₃ à 400 MHz et par leur pouvoir rotatoire spécifique.

### Composé 12a

### (2S,3R)-N-benzyloxycarbonyl-3-Phénéthyl-aziridine-2-carboxylate d'éthyle

Rdt : 93%. CCM (cyclohexane : acétate d'éthyle, 70 : 30) : R*_{f}* = 0,47. [α]²⁰_{D} = +30,2° (c = 1,026, MeOH). HPLC (70%B) : Rt = 13,7 min. RMN ¹H (CDCl₃): 1,25 (t, 3H, COOCH₂CH₃), 1,87 (m, 2H, CH₂ C-NH), 2,7-2,9 (m, 4H, CH₂Phe, CHCH₂, CHCOOEt), 4,15 (q, 2H, COOCH₂CH₃), 5,15 (2d, 2H, OCH₂Phe), 7,15-7,3 (m, 5H, Ph), 7,3-7,4 (m, 5H, Ph).

### Composé 12b

### (2R,3R)-N-benzyloxycarbonyl-3-Phénéthyl-aziridine-2-carboxylate d'éthyle

Rdt: 91%. CCM (cyclohexane : acétate d'éthyle, 80: 20): R*_{f}* = 0,27. [α]²⁰_{D} = +42,49° (c = 1,024, MeOH). HPLC (80%B) : Rt = 6,6 min. RMN ¹H (CDCl₃) : 1,3 (t, 3H, COOCH₂CH₃), 1,9-2 (m, 2H, CH₂C-NH), 2,75 (m, 2H, CH₂Phe, 2,85 (m, 1H, CHCH₂), 3,2 (d, 1H, CHCOOEt), 4,2 (q, 2H, COOCH₂CH₃), 5,15 (m, 2H, OCH₂Phe), 7,1-7,3 (m, 5H, Ph), 7,3-7,4 (m, 5H, Ph).

### Composé 12c

### (2R,3S)-N-benzyloxycarbonyl-3-Phénéthyl-aziridine-2-carboxylate d'éthyle

Rdt: 90%. CCM (cyclohexane : acétate d'éthyle, 70 : 30) : R*_{f}* = 0,47. [α]²⁰_{D} = -28,6° (c = 1,05, MeOH). HPLC (70%B) : Rt = 13,84 min. RMN ¹H (CDCl₃) : 1,25 (t, 3H, COOCH₂CH₃), 1,87 (m, 2H, CH₂ C-NH), 2,7-2,9 (m, 4H, CH₂Phe, CHCH₂, CHCOOEt), 4,15 (q, 2H, COOCH₂CH₃), 5,15 (2d, 2H, OCH₂Phe), 7,15-7,3 (m, 5H, Ph), 7,3-7,4 (m, 5H, Ph).

### Composé 12d

### (2S,3S)-N-benzyloxycarbonyl-3-Phénéthyl-aziridine-2-carboxylate d'éthyle

Rdt: 81%. CCM (cyclohexane: acétate d'éthyle, 70: 30) : R*_{f}* = 0,49. [α]²⁰_{D} = -39,58° (c = 0,53, MeOH). HPLC (70%B) : Rt = 11,43 min RMN ¹H (CDCl₃) : 1,3 (t, 3H, COOCH₂CH₃), 1,9 - 2 (m, 2H, CH₂C-NH), 2,75 (m, 2H, CH₂Phe, 2,85 (m, 1H, CHCH₂), 3,2 (d, 1H, CHCOOEt), 4,2 (q, 2H, COOCH₂CH₃), 5,15 (m, 2H, OCH₂Phe), 7,1-7,3 (m, 5H, Ph), 7,3-7,4 (m, 5H, Ph).

### Préparation 13 : synthèse des composés 2e₁₋₄

A une solution refroidie à 0°C de 0,65 g (1,8 mmol, 1 équivalent) du composé 12, dans 11 ml (6 ml/mmol) de dichlorométhane anhydre, on ajoute successivement 1,0 g, 1 ml (6,4 mmol, 3,6 équivalents) de 4-méthoxybenzylmercaptan puis au goutte à goutte 0,7 ml (5,5 mmol, 3 équivalents) de BF₃ éthérate. Le mélange est agité 24 h à 4°C. Après addition de 18 ml de NaHCO₃ 10% (10 ml/mmol) et de 18 ml de dichlorométhane, la phase organique est séparée, séchée sur Na₂SO₄ et concentrée sous pression réduite. L'huile obtenue est purifiée par filtration sur silice. On obtient une huile incolore.

Les composés 2e₁₋₄ ont été caractérisés par CCM sur un support de silice avec un éluant cyclohexane : acétate d'éthyle, par leur spectre ¹H RMN dans CDCl₃ à 400 MHz et par leur pouvoir rotatoire spécifique.

### Composé 2e₁

### (2S,3R)-2-Benzyloxycarbonylamino-3-(4-méthoxy-benzylsulfanyl)-5-phényi-pentanoate d'éthyle

Rdt : 38%. CCM (n-heptane : acétate d'éthyle, 75 : 25) : R*_{f}* = 0,25. [α]²⁰_{D} = -96,1° (c = 0,282, MeOH). HPLC (70%B) : Rt = 23 min RMN ¹H (CDCl₃) : 1,15 (t, 3H, COOCH₂CH₃), 1,75 (m, 1H, CH₂CHS), 1,8 (m, 1H, CH₂CHS), 2,55 (m, 1H, CHS), 2,76 (m, 1H, CH₂Phe), 2,83 (m, 1H, CH₂Phe), 3,7 (2d, 2H, SCH₂), 3,8 (s, 3H, OCH₃), 3,96-4,17 (m, 2H, COOCH₂CH₃), 4,65 (dd, 1H, CHCOOEt), 5,13 (m, 2H, OCH₂Phe), 5,43 (du, 1H, NH), 6,83 (d, 2H, Ar), 6,96 (d, 2H, Ar), 7,1-7,25 (m, 5H, Ph) 7.,3-7,4 (m, 5H, Ph).

### Composé 2e₂

### (2S,3S)-2-Benzyloxycarbonylamino-3-(4-méthyl-benzylsulfanyl)-5-phényl-pentanoate d'éthyle

Rdt: 79%. CCM (cyclohexane : acétate d'éthyle, 70: 30): R*_{f}* = 0,36. [α]²⁰_{D} = -19° (c = 1,004, MeOH). HPLC (80%B) : Rt = 11,8 min RMN ¹H (CDCl₃) : 1,25 (t, 3H, COOCH₂CH₃), 1,77 (m, 1H, CH₂CHS), 1,93 (m, 1H, CH₂CHS), 2,6 -2,77 (m, 2H, CH₂Phe), 3,1 (m, 1H, CHS), 3,57 (s, 2H, SCH₂), 3,8 (s, 3H, OCH₃), 4,07-4,23 (2m, 2H, COOCH₂CH₃), 4,7 (d, 1H, CHCOOEt), 5,13 (s, 2H, OCH₂Phe), 5,55 (d, 1H, NH), 6,8 (d, 2H, Ar), 7,1 (d, 2H, Ar), 7,15-7,4 (m, 10H, Ph).

### Composé 2e₃

### (2R,3S)-2-Benzyloxycarbonylamino-3-(4-méthyl-benzylsulfanyl)-5-phényl-pentanoate d'éthyle

Rdt: 43%. CCM (cyclohexane: acétate d'éthyle, 80: 20): R*_{f}* = 0,24. [α]²⁰_{D} = +96,0° (c = 0,3, MeOH). HPLC (70%B) : Rt = 23 min. RMN ¹H (CDCl₃) : 1,15 (t, 3H, COOCH₂CH₃), 1,75 (m, 1H, CH₂CHS), 1,8 (m, 1H, CH₂CHS), 2,55 (m, 1H, CHS), 2,76 (m, 1H, CH₂Phe), 2,83 (m, 1H, CH₂Phe), 3,7 (2d, 2H, SCH₂), 3,8 (s, 3H, OCH₃), 3,96-4,17 (m, 2H, COOCH₂CH₃), 4,65 (dd, 1H, CHCOOEt), 5,13 (m, 2H, OCH₂Phe), 5,43 (d, 1H, NH), 6,83 (d, 2H, Ar), 6,96 (d, 2H, Ar), 7,1-7,25 (m, 5H, Ph) 7,3-7,4 (m, 5H, Ph).

### Composé 2e₄

### (2R,3R)-2-Benzyloxycarbonylamino-3-(4-méthyl-benzylsulfanyl)-5-phényl-pentanoate d'éthyle

Rdt: 64%. CCM (cyclohexane: acétate d'éthyle, 70: 30): R*_{f}* = 0,36. [α]²⁰_{D} = +20,2° (c = 0,5, MeOH). HPLC (70%B) : Rt = 25,81 min. RMN ¹H (CDCl₃) : 1,25 (t, 3H, COOCH₂CH₃), 1,77 (m, 1H, CH₂CHS), 1,93 (m, 1H, CH₂CHS), 2,6 -2,77 (m, 2H, CH₂Phe), 3,1 (m, 1H, CHS), 3,57 (s, 2H, OCH₂), 3,8 (s, 3ff, OCH₃), 4,07-4,23 (2m, 2H, COOCH₂CH₃), 4,7 (d, 1H, CHCOOEt), 5,13 (s, 2H, OCH₂Phe), 5,55 (d, 1H, NH), 6,8 (d, 2H, Ar), 7.1 (du, 2H, Ar), 7,15-7,4 (m, 10H, Ph).

### Préparation 14 : synthèses des composés 3e₁₋₄

A une solution de diéthoxyphosphorylméthanesulfonate de néopentyl (2,0 équivalents) dans du THF anhydre (4,5 ml/mmol) on ajoute goutte à goutte à -78°C, une solution de n-butyllithium 1,6 M dans l'hexane (2 équivalents). Après 30 min. sous agitation, on ajoute une solution du composé 2 (1 équivalent) dans du THF anhydre (1 ml/mmol) suivi de l'introduction goutte à goutte d'une solution d'hydrure de diisobutylaluminium 1,6 M dans le toluène (2 équivalents). Le mélange réactionnel est maintenu à -78°C 4h, on laisse ensuite revenir à température ambiante une nuit. Les solvants sont éliminés sous pression réduite, le résidu est additionné de 10 ml/mmol d'éther et de 5 ml/mmol d'HCl 2N. Le mélange est agité 30 min, la phase organique est séparée, la phase aqueuse est extraite deux fois avec un volume d'éther équivalent. Les phases organiques sont réunies, séchées sur Na₂SO₄ et concentrées sous pression réduite. L'huile obtenue est purifiée par chromatographie sur silice. Après élimination des solvants le produit 3 est obtenu sous forme d'une huile.

Les composés 3e₁₋₄ ont été caractérisés par CCM sur un support de silice avec un éluant cyclohexane: acétate d'éthyle, par leur spectre ¹H RMN dans CDCl₃ à 400 MHz et par leur pouvoir rotatoire spécifique.

### Composé 3e₁

### (3R,4S)-3-Benzyloxycarbonylamino-4-(4-méthoxy-benzylsulfanyl)-6-phényl-hex-1-ène-1-sulfonate de 2,2-diméthylpropyle

Rdt: 27%. CCM (cyclohexane : acétate d'éthyle, 80: 20): R*_{f}* = 0,17. [α]²⁰_{D} = -39,2° (c = 1,1, MeOH). HPLC (80%B) : Rt = 15,33 min. RMN ¹H (CDCl₃) : 0,95 (s, 9H, 3x CH₃), 1,8 (m, 1H, CH₂CHS), 1,93 (m, 1H, CH₂CHS), 2,65 (m, 2H, CH₂Phe), 2,83 (m, 1H, CHS), 3,6-3,7 (m, 2H, SO₃CH₂), 3,7 (s, 2H, SCH₂), 3,75 (s, 3H, OCH₃), 4,63 (m, 1H, CHNH), 5,07 (m, 3H, OCH₂phe, NH), 6,3 (d, 1H, C=CHSO₃), 6,75 (dd, 1H, CH=CSO₃), 6,8 (d, 2H, Ar), 7,07 (d, 2H, Ar), 7,1-7,5 (m, 10H, Ph).

### Composé 3e₂

### (3S,4S)-3-Benzyloxycarbonylamino-4-(4-méthoxy-benzylsulfanyl)-6-phényl-hex-1-ène-1-sulfonate de 2,2-diméthylpropyle

Rdt : 54%. CCM (cyclohexane : acétate d'éthyle, 80 : 20) : R*_{f}* = 0,37. [α]²⁰_{D} = -56,5° (c = 1,01, MeOH). HPLC (80%B) : Rt = 16,37 min. RMN ¹H (CDCl₃) : 0,95 (s, 9H, 3x CH₃), 1,7 -1,9 (m, 2H, CH₂CHS), 2,5 -2,8 (m, 3H, CH₂Phe, CHS), 3,63 (s, 2H, SO₃CH₂), 3,7 - 3,85 (2d, 2H, SCH₂, s, 3H, OCH₃), 4,75 (m, 1H, CHNH), 5,1 (m, 2H, OCH₂Phe), 5,2 (d, 1H, NH), 6,35 (d, 1H, C=CHSO₃), 6,75 - 6,9 (m, 3H, CH=CSO₃, Ar), 7 (d, 2H, Ar), 7,15-7,4 (m, 10H, Ph).

### Composé 3e₃

### (3S,4R)-3-Benzyloxycarbonylamino-4-(4-méthoxy-benzylsulfanyl)-6-phényl-hex-1-ène-1-sulfonate de 2,2-diméthylpropyle

Rdt: 20%. CCM (cyclohexane : acétate d'éthyle, 80 : 20) : R*_{f}* = 0,17 [α]²⁰_{D} = + 38,5° (c = 1,0, MeOH). HPLC (80%B) : Rt = 15,33 min. RMN ¹H (CDC13) : 0,95 (s, 9H, 3x CH₃), 1,8 (m, 1H, CH₂CHS), 1,93 (m, 1H, CH₂CHS), 2,65 (m, 2H, CH₂Phe), 2,83 (m, 1H, CHS), 3,6-3,7 (m, 2H, SO₃CH₂), 3,7 (s, 2H, SCH₂), 3,75 (s, 3H, OCH₃), 4,63 (m, 1H, CHNH), 5,07 (m, 3H, OCH₂Phe, NH), 6,3 (d, 1H, C=CHSO₃), 6,75 (dd, 1H, CH=CSO₃), 6,8 (d, 2H, Ar), 7,07 (d, 2H, Ar), 7,1-7,5 (m, 10H, Ph).

### Composé 3e₄

### (3R,4R)-3-Benzyloxycarbonylamino-4-(4-méthoxy-benzylsulfanyl)-6-phényl-hex-1-ène-1-sulfonate de 2,2-diméthylpropyle

Rdt: 35%. CCM (cyclohexane : acétate d'éthyle, 80 : 20) : R*_{f}* = 0,37. [α]²⁰_{D} = +55,4° (c = 0,95, MeOH). HPLC (80%B) : Rt = 16,37 min. RMN ¹H (CDCl₃) : 0,95 (s, 9H, 3x CH₃), 1,7 -1,9 (m, 2H, CH₂CHS), 2,5 -2,8 (m, 3H, CH₂Phe, CHS), 3,63 (s, 2H, SO₃CH₂), 3,7 - 3,85 (2d, 2H, SCH₂, s, 3H, OCH₃), 4,75 (m, 1H, CHNH), 5,1 (m, 2H, OCH₂Phe), 5,2 (d, 1H, NH), 6,35 (d, 1H, C=CHSO₃), 6,75 - 6,9 (m, 3H, CH=CSO₃, Ar), 7 (d, 2H, Ar), 7,15-7,4 (m, 10H, Ph).

### Préparation 15 : synthèse des composés 4e₁₋₄

A une solution d'un composé 3 (1 équivalent) dans de l'éthanol absolu (5 ml/mmol) on ajoute du borohydrure de sodium (1 équivalent). Le mélange réactionnel est agité à 25°C une nuit. Les solvants sont éliminés sous pression réduite, le résidu est additionné de 10 ml/mmol d'acétate d'éthyle et de 5 ml/mmol d'eau. La phase organique est séparée, la phase aqueuse est extraite deux fois avec un volume d'acétate d'éthyle équivalent. Les phases organiques sont réunies, séchées sur Na₂SO₄ et concentrées sous pression réduite. L'huile obtenue est purifiée par HPLC semi-préparative. Après élimination des solvants le produit 16 est obtenu sous forme d'une huile.

Les composés 4e₁₋₄ ont été caractérisés par CCM sur un support de silice avec un éluant cyclohexane: acétate d'éthyle, par leur spectre ¹H RMN dans CDCl₃ à 400 MHz et par leur pouvoir rotatoire spécifique.

### Composé 4e₁

### (3R,4S)-3-Benzyloxycarbonylamino-4-(4-méthoxy-benzylsulfanyl)-6-phényl-hexane-1-sulfonate de 2,2-diméthylpropyle:

Rdt: 60%. CCM (cyclohexane: acétate d'éthyle, 80: 20) : R*_{f}* = 0,15. [α]²⁰_{D} =-33,7° (c = 0,4, MeOH). HPLC (80%B) : Rt = 14,37 min. RMN ¹H (CDCl₃) : 0,97 (s, 9H, 3x CH₃), 1,8(m, 1H, CH₂CHS), 1,9 (m, 1H, CH₂CHS) ), 1,9-2,05 (m, 2H, CH₂C-NH), 2,6 (m, 2H, CH₂Phe), 2,8 (m, 1H, CHS), 3,07 (m, 2H, CH₂SO₃), 3,63 (m, 2H, SO₃CH₂), 3,75 (s, 3H, OCH₃), 3,83 (s, 2H, SCH₂), 3,93 (m, 1H, CHNH), 4,97 (d, 1H, NH), 5,05 (m, 2H, OCH₂Phe), 6,8 (d, 2H, Ar), 7,07 (d, 2H, Ar), 7,1-7,4 (m, 10H, Ph).

### Composé 4e₂

### (3S,4S)-3-Benzyloxycarbonylamino-4-(4-méthoxy-benzylsutfanyl)-6-phényl-hexane-1-sulfonate de 2,2-diméthylpropyle

Rdt: 63%. CCM (cyclohexane : acétate d'éthyle, 80: 20) : R*_{f}* = 0,12. [α]₂₀^{D} = -36,4° (c = 0,936, MeOH), HPLC (80%B) : Rt = 15,2 min. RMN ¹H CDCl₃ : 1 (s, 9H, 3x CH₃), 1,75(m, 1H, CH₂CHS), 1,8-2,1 (m, 3H, CH₂CHS, CH₂C-NH), 2,5 (m, 1H, CHS), 2,6 (m, 1H, CH₂Phe), 2,73 (m, 1H, CH₂Phe), 3 (m. 2H, CH₂SO₃), 3,67(m, 2H, SO₃CH₂), 3,8 (s, 3H, OCH₃), 3,83 (s, 2H, SCH₂), 3,97 (m, 1H, CHNH). 4,9 (d, 1H, NH), 5.1 (m. 2H, OCH₂Phe), 6,83 (d, 2H, Ar), 7,05 (d, 2H, Ar), 7,1 - 7,4 (m, 10H, Ph).

### Composé 4e₃

### (3S,4R),3-Benzyloxycarbonylamino4-(4-méthoxy-benzylsulfanyl)-6-phényl-hexane-1-sulfonate de 2,2-diméthylpropyle

Rdt: 83%. CCM (cyclohexane : acétate d'éthyle, 80 : 20) : R*_{f}* = 0,15. [α]²⁰_{D} = +31,7° (c= 0.4, MeOH). HPLC (80%B) : Rt =14,25 min. RMN ¹H (CDCl₃) : 0,97 (s, 9H, 3x CH₃), 1,8(m, 1H, CH₂CHS), 1,9 (m, 1H, CH₂CHS) ), 1,9-2,05 (m, 2H, CH₂C-NH), 2.6 (m, 2H, CH₂Phe), 2,8 (m, 1H, CHS), 3.07 (m, 2H, CH₂SO₃), 3,63 (m, 2H, SO₃CH₂), 3,75 (s, 3H, OCH₃), 3,83 (s, 2H, SCH₂) , 3,93 (m, 1H, CHNH), 4,97 (d, 1H, NH), 5,05 (m, 2H, OCH₂Phe), 6,8 (d, 2H, Ar), 7,07 (d, 2H, Ar), 7,1-7,4 (m, 10H, Ph).

### Composé 4e₄

### (3R, 4R) -3-Benzyloxycarbonylamino-4-(4-méthoxy-benzylsulfanyl)-6-phényl-hexane-1-suifonate de 2,2-diméthytpropyle

Rdt: 67%. CCM (cyclohexane : acétate d'éthyle, 80: 20) : R*_{f}* = 0,12 [α]₂₀^{D} = + 35,8° (c = 0,8, MeOH). HPLC (80%B) : Rt =15,0 min. RMN ¹H (CDCl₃) : 1 (s, 9H, 3x CH₃), 1,75(m, 1H, CH₂CHS), 1.8-2,1 (m, 3H, CH₂CHS, CH₂C-NH), 2,5 (m, 1H, CHS), 2,6 (m, 1H, CH₂Phe), 2,73 (m, 1H, CH₂Phe), 3 (m, 2H, CH₂SO₃), 3,67(m, 2H, SO₃CH₂), 3,8 (s, 3H, OCH₃). 3.83 (s, 2H. SCH₂) , 3,97 (m, 1H, CHNH), 4,9 (d, 1H, NH). 5,1 (m, 2H, OCH₂Phe), 6.83 (d. 2H, Ar), 7,05 (d, 2H, Ar), 7,1 - 7,4 (m, 10H, Ph).

### Préparation 16 : synthèse des compos- 5e₁₋₄

Au composé 4 (1 équivalent) on ajoute de l'anisole (5 équivalents) et de l'acide trifluoroacétique (7 ml/mmol). Le mélange réactionnel est porté a reflux sous argon 16 heures. Le solvant est éliminés sous pression réduite. Le résidu est suspendu dans 5 ml/mmol de cyclohexane qui est ensuite éliminé sous pression réduite. Cette opération est répétée deux fois afin d'éliminer les traces d'acide trifluoroacétique (TFA). L'huile obtenue est additionnée d'éther, le composé 5 précipite et est séché sous pression réduite après filtration. Le composé 5 est ensuite purifié par HPLC semi-préparative. Après lyophilisation on obtient un solide incolore.

Les composés 5e₁₋₄ ont été caractérisés par leur spectre ¹H RMN dans DMSO-D₆ + TFA à 400 MHz et par leur pouvoir rotatoire spécifique.

### Composé 5e₁

### (3R,4S)-Acide 3-amino-4-mercapto-6-phényl-hexane-1-sulfonique

Rdt : 54%. [α]₂₀^{D} = -19,8 (c = 0,77, H₂O). HPLC (gradient 10-90%B en 30 min): Rt = 12,66 min. ES-MS[M+Na]⁺ 312. RMN ¹H (DMSO-D₆+TFA) : 1,65 (m, 1H, CH₂CHS), 1,73-2 (m, 3H, CH₂CHS, CH₂C-NH₂), 2,6 (m, 3H, CH₂ SO₃H, CH₂Phe) 2,8 (m, 1H, CH₂Phe), 2,93 (m, 1H, CHSH), 3,47 (m, 1H, CHNH₂), 7,1-7,3 (m, 5H, Ph), 7,9 (s, 3H, NH₃⁺)

### Composé 5e₂

### (3S,4S)-Acide 3-amino-4-mercapto-6-phényl-hexane-1-sulfonique

Rdt : 49%. [α]₂₀^{D} = -33,9 (c = 0,44, H₂O). HPLC (gradient 10-90%B en 30 min) : Rt = 12,68 min. ES-MS[M+Na]⁺ 312. RMN ¹H (DMSO-D₆+TFA) : 1,65 (m, 1H, CH₂CHS), 1,87 (m, 1H, CH₂CHS), 1,97 (m, 2H, CH₂C-NH₂), 2,6 (m, 3H, CH₂ SO₃H, CH₂Phe) 2,8 (m, 1H, CH₂Phe), 2,93 (m, 1H, CHSH), 3,4 (m, 1H, CHNH₂), 7 -7,3 (m, 5H, Ph), 7,9 (s, 3H, NH₃⁺).

### Composé 5e₃

### (3S,4R)-Acide 3-amino-4-mercapto-6-phényl-hexane-1-sulfonique

Rdt : 40%. [α]20D = = +22,4 (c =0,1, H₂O)° HPLC (gradient 10-90%B en 30 min) : Rt = 12,63 min. ES-MS[M+Na]⁺ 312,17. RMN ¹H (DMSO-D₆+TFA) : 1,65 (m, 1H, CH₂CHS), 1,73-2 (m, 3H, CH₂CHS, CH₂C-NH₂), 2,6 (m, 3H, CH₂ SO₃H, CH₂Phe) 2,8 (m, 1H, CH₂Phe), 2,93 (mu, 1H, CHSH), 3,47 (m. 1H, CHCH₂). 7,1-7,3 (m, 5H, Ph), 7,9 (s, 3H, NH₃⁺)

### Composé.5e₄

### (3R,4R)-Acide 3-amino-4-mercapto-6-phényl-hexane-1-sulfonique

Rdt : 53%. [α]20D = = +30,5 (c =0,1, H₂O). HPLC (gradient 10-90%B en 30 min) : Rt = 12,67 min. ES-MS[M+Na]⁺ 312 14. RMN ¹H (DMSO-D₆+TFA) : 1,65 (m, 1H, CH₂CHS), 1,87 (m, 1H, CH₂CHS), 1,91 (m, 2H, CH₂C-NH₂), 2,6 (m, 3H, CH₂ SO₃H, CH₂Phe) 2,8 (m, 1H, CH₂Phe), 2,93 (m, 1H, CHSH), 3,4 (m, 1H, CHNH₂), 7 -7,3 (m, 5H, Ph), 7,9 (s, 3H, NH₃⁺).

### Exemple 1 :Synthèse du composé 5e dimère

Au composé 5e (1 équivalent) en solution dans de l'éthanol (0,1 ml/mmol) on additionne goutte à goutte sous agitation à 25°C une solution aqueuse 0,1 M d'iode jusqu'à persistance de la coloration. Après concentration sous pression réduite, l'huile cristallise en présence d'éther. Le produit est séché sous pression réduite après filtration.

Le composé 5e dimère a été caractérisé par son spectre ¹H RMN dans DMSO-D₆ à 400 MHz et par spectrométrie de masse électrospay.

### Composé 5e dimère

### Acide 4,4'-dithiobis-(3,3'-amino-6,6'-phényl-1-1'-hexane sulfonique)

Rdt 85%. RMN ¹H (DMSO-D₆): 1,6 (m, 2H, CH₂-CH-S), 1,7-2,0 (m, 6H, CH₂-CH₂-CH-S, CH₂CH₂-SO₃), 2,6-2,8 (m, 8H, CH₂-CH-S, CH₂CH₂-SO₃, CH₂-CH-S), 2,9-3,2 (m, 2H, CHS), 3,6 (m, 2H, CH-N), 7,1 (m, 10 H, H aromatique), 7,9-8,1 (m, 6H, NH₃⁺). ES⁻ : 275 M-H⁺. ES+ : 599 M+Na⁺

### Exemple 2 : détermination des constantes d'inhibition des composés vis-à-vis de l'APA.

Les composés 5 ont été testés *in vitro* sur l'aminopeptidase A recombinante pour déterminer leur affinité pour l'APA.

Le dosage de l'activité APA est basé sur le protocole de Goldbarg adapté à l'échelle de dosage sur microplaques (Pro Bind ® 3915) (Chauvel et al., Je. Med. Chem., 1994, 37.1339-1346).

### Principe

*In vitro,* en présence d'ions calcium, l'APA hydrolyse l'α-L-glutamyl-β-napthtylamide (gluβNa) en glutamate et β**-**naphtylamine (βNa). Une réaction de diazotation en milieu acide permet de révéler le β-naphtylamine par formation d'un complexe de couleur violette : une mesure de spectrophotométrie permet alors de connaître la quantité de complexe formé et, par référence à une gamme étalon réalisée avec des concentrations croissantes de β**-**naphtylamine, d'en déduire l'activité enzymatique de l'échantillon.

### Réactifs

Le substrat GluβNa et le β-naphtylamine (Bachem) sont solubilisés dans le diméthylsulfoxyde et l'HCl 0,1 N respectivement, et conservés à - 20 °C à la concentration de 10⁻² M. La réaction de diazotation se fait en présence de nitrite de sodium (87 mM), de sulfamate d'ammonium (130 mM) et de dichlorhydrate de N-(1-naphtyl)éthylènediamine (23 mM).

### Réaction enzymatique

La réaction a lieu à pH 7,4 en tampon Tris-HCl 50 mM, en présence de calcium (CaCl₂, 4 mM) ; l'échantillon à doser est incubé à 37 °C en présence de substrat (GluβNa, 200 µM) et en absence ou en présence de différentes concentrations de l'inhibiteur à tester dans un volume final de 100 µl. La réaction est arrêtée par addition de 10 µl d'HCl 3N. Une gamme étalon de β-naphtylamine en milieu acide (ajouter 10 µl d'HCl 0,1 N) est effectuée en parallèle.

### Révélation du produit formé

On ajoute dans chaque puits :
- 25 µl de nitrite de sodium (mélanger, attendre 5 min à température ambiante),
- 50 µl de sulfamate d'ammonium (agiter, attendre 5 min à température ambiante), puis
- 25 µl de dichrorhydrate de N-(1-naphtyl)éthylènediamine 23 mM (mélanger, attendre la stabilisation de la couleur violette environ 30 min à 37 °C).

L'absorbance est ensuite mesurée à 540 nm.

Le composé EC 33 (acide (S) 3 -amino 4-mercaptobutylsulfonique) décrit dans la demande WO 99/36066), monomère du composé RB 150 (acide 4,4'-dithiobis 3-amino butane-1-sulfonique) décrit dans la demande WO 2004/007441, a servi de composé de référence.

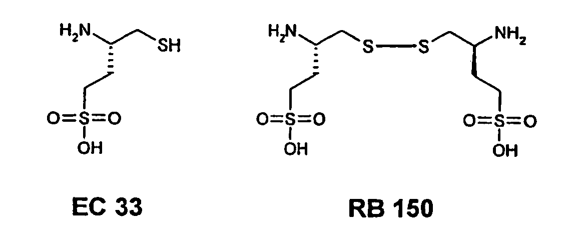

Les résultats sont rapportés dans les tableaux 1 et 2 ci-après.

**Tableau 1**

| composés | EC 33 | 5a | 5b | 5c | 5d | 5e | 5f | 5g | 5h |
|---|---|---|---|---|---|---|---|---|---|
| Ki (µM) | 0,304 | >100 | 8,00 | 4,00 | 0,380 | 0,08 | 0,092 | 0,096 | 0,110 |

Les résultats montrent que les composés 5d, 5e, 5f, 5g et 5h qui possèdent une chaîne R₂ alkyle en C₁ ou C₂ substituée par un groupe phényle éventuellement substitué présentent une activité inhibitrice du même ordre ou supérieure à celle du composé de référence.

**Tableau 2**

| composés | EC 33 | 5e1 | 5e2 | 5e3 | 5e4 |
|---|---|---|---|---|---|
| Ki (µM) | 0,304 | 1,92 | 0,03 | 1,04 | 0,84 |

Les résultats montrent que le compose 5e2, de configuration: (S),(S), présente l'activité inhibitrice de l'APA la plus élevée, supérieure d'un facteur 10 à celle du composé de référence.

## Revendications

1. Composé **caractérisé en ce qu'**il répond à la formule (1) 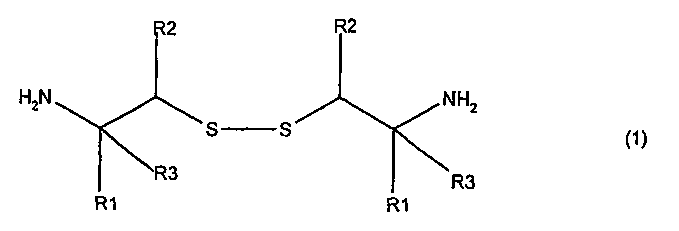 dans laquelle
chaque groupement R¹ est identique à l'autre groupement R¹ et représente :
- un groupe alkyle en C₁ à C₆, alcényle en C₁ à C₆ ou alcynyle en C₁ à C₆,
- un groupe (CH₂)ₙbenzyle dans lequel n est égal à 0 ou 1,
- un groupe (CH₂)ₘ(cycloalkyle en C₃ à C₆) dans lequel m est égal à 0 ou 1, chacun des groupes alkyle, alcényle, alcynyle, benzyle ou cycloalkyle étant substitué par un ou deux groupe(s) représenté(s) par le groupement A ; le groupement A représente :
- un groupe carboxylate COOH ou COOR, R représentant un groupe alkyle en C₁ à C₆ ou CH₂phényle ;
- un groupe sulfonate SO₃H ou SO₃R', R' représentant un groupe alkyle en C₁ à C₆ ou CH₂phényie ;
- un groupe phosphonate PO₃H₂ ou PO₃R₂"R"', R" et R"' représentant indépendamment H, un groupe alkyle en C₁ à C₆ ou CH₂phényle ;
• chaque groupement R² est identique à l'autre groupement R² et représente un groupe alkyle en C₁ à C₆, alcényle en C₂ à C₆ ou alcynyle en C₂ à C₆, chaque groupe alkyle, alcényle ou alcynyle étant libre ou substitué par le groupement B ;
• le groupement B représente :
- un groupe carboxylate, COOH ou COOR', R' représentant un groupe alkyle en C₁ à C₆ ou CH₂phényle ;
- un groupe phényle libre ou substitué par un ou plusieurs radicaux choisis parmi un atome d'halogène, un radical hydroxyle éventuellement protégé, un groupe alkyle en C₁ à C₄, un groupe cyano, un groupe carboxyle libre, salifié, estérifié ou un groupe amide ;
• chaque groupement R³ est identique à l'autre groupement R³ et représente un atome d'hydrogène.

2. Composé selon la revendication 1, **caractérisé en ce que** R¹ est choisi entre des groupes alkyle en C₁ à C₆, alcényle en C₂ à C₆ et benzyle, chacun de ces groupes étant substitué par un ou deux groupe(s) représenté(s) par le groupement A tel que défini dans la revendication 1.

3. Composé selon l'une des revendications 1 ou 2, **caractérisé en ce que** R² est choisi entre un groupe alkyle en C₁ à C₆ et un groupe alcényle en C₂ à C₆, chacun de ces groupes pouvant être substitué par un ou deux groupe(s) représenté(s) par le groupement B tel que défini dans la revendication 1.

4. Composé selon l'une quelconque des revendications 1 à 3, **caractérisé en ce que** R¹ représente un groupe éthyle substitué par un groupe sulfonique, phosphonique ou carboxylique, libre, salifié ou estérifié et R² représente un groupe éthyle substitué par un groupe phényle libre ou substitué.

5. Composé selon l'une quelconque des revendications 1 à 4, **caractérisé en ce qu'**il s'agit de l'acide 4,4'-dithiobis-(3,3'-amino-6,6'-phényl-1-1'-hexane sulfonique).

6. Composé selon la revendication 5, **caractérisé en ce qu'**il s'agit de l'acide 4(S),4'(S),3(S), 3'(S)-4'-dithiobis-(3;3'-amino-6,6'-phényl-1-1'-hexane sulfonique).

7. Composé selon l'une quelconque des revendications 1 à 6, **caractérisé en ce qu'**il est destiné à être utilisé en thérapeutique.

8. Composition pharmaceutique, **caractérisée en ce qu'**elle comprend un composé selon l'une quelconque des revendications 1 à 6.

9. Utilisation: d'un composé selon l'une quelconque des revendications 1 à 6, pour la préparation d'un médicament en tant qu'inhibiteur sélectif à l'égard de l'aminopeptidase A.

10. Composé selon l'une quelconque des revendications 1 à 6, pour le traitement de l'hypertension artérielle et des maladies directement et indirectement liées.

11. Composé selon l'une quelconque des revendications 1 à 6, pour le traitement d'une maladie choisie parmi l'hypertension artérielle primaire et secondaire, un ictus, l'ischémie myocardique, l'insuffisance cardiaque et l'insuffisance rénale, l'infarctus du myocarde, une maladie vasculaire périphérique, la protéinurie diabétique, le syndrome X, le glaucome, les maladies neurodégénératives et les troubles de la mémoire.

12. Composé selon l'une quelconque des revendications 1 à 6, pour le traitement de pathologies ischémiques et tumorales dans lesquelles l'aminopeptidase A est impliquée.

## Claims

1. Compound **characterized in that** it corresponds to formula (1) 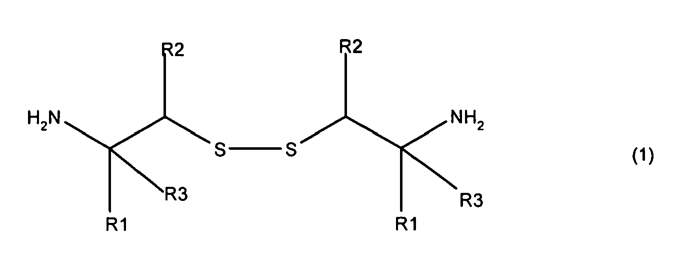 in which
• each group R¹ is identical to the other group R¹ and represents:
- a C₁ to C₆ alkyl, C₂ to C₆ alkenyl or C₂ to C₆ alkynyl group,
- a (CH₂)ₙbenzyl group in which n is equal to 0 or 1,
- a (CH₂)ₘ(C₃ to C₆ cycloalkyl) group in which m is equal to 0 or 1,
each of the alkyl, alkenyl, alkynyl, benzyl or cycloalkyl groups being substituted with one or two group(s) represented by the group A;
• the group A represents:
- a carboxylate group COOH or COOR, R representing a C₁ to C₆ alkyl or CH₂phenyl group;
- a sulfonate group SO₃H or SO₃R', R' representing a C₁ to C₆ alkyl or CH₂phenyl group;
- a phosphonate group PO₃H₂ or PO₃R₂"R"', R" and R"' independently representing H, or a C₁ to C₆ alkyl or CH₂phenyl group;
• each group R² is identical to the other group R² and represents a C₁ to C₆ alkyl, C₂ to C₆ alkenyl or C₂ to C₆ alkynyl group, each alkyl, alkenyl or alkynyl group being free or substituted with the group B;
• the group B represents:
- a carboxylate group, COOH or COOR', R' representing a C₁ to C₆ alkyl or CH₂phenyl group;
- a phenyl group that is free or substituted with one or more radicals chosen from a halogen atom, an optionally protected hydroxyl radical, a C₁ to C₄ alkyl group, a cyano group, a free, salified or esterified carboxyl group or an amide group;
• each group R³ is identical to the other group R³ and represents a hydrogen atom.

2. Compound according to Claim 1, **characterized in that** R¹ is chosen from C₁ to C₆ alkyl, C₂ to C₆ alkenyl and benzyl groups, each of these groups being substituted with one or two group(s) represented by the group A as defined in Claim 1.

3. Compound according to either of Claims 1 and 2, **characterized in that** R² is chosen from a C₁ to C₆ alkyl group and a C₂ to C₆ alkenyl group, it being possible for each of these groups to be substituted with one or two group(s) represented by the group B as defined in Claim 1.

4. Compound according to any one of Claims 1 to 3, **characterized in that** R¹ represents an ethyl group substituted with a sulfonic group, a phosphonic group or a carboxylic group, that is free, salified or esterified, and R² represents an ethyl group substituted with a free or substituted phenyl group.

5. Compound according to any one of Claims 1 to 4, **characterized in that** it is 4,4'-dithiobis-(3,3'-amino-6,6'-phenyl-1,1'-hexanesulfonic) acid.

6. Compound according to Claim 5, **characterized in that** it is 4(S),4'(S),3(S),3'(S)-4'-dithiobis-(3,3'-amino-6,6'-phenyl-1,1'-hexanesulfonic) acid.

7. Compound according to any one of Claims 1 to 6, **characterized in that** it is intended to be used in therapeutics.

8. Pharmaceutical composition, **characterized in that** it comprises a compound according to any one of Claims 1 to 6.

9. Use of a compound according to any one of Claims 1 to 6, for preparing a medicinal product as a selective inhibitor with regard to aminopeptidase A.

10. Compound according to any one of Claims 1 to 6, for the treatment of arterial hypertension and of directly and indirectly related diseases.

11. Compound according to any one of Claims 1 to 6, for the treatment of a disease chosen from primary or secondary arterial hypertension, an ictus, myocardial ischemia, cardiac insufficiency and renal insufficiency, myocardial infarction, a peripheral vascular disease, diabetic proteinuria, syndrome X, glaucoma, neurodegenerative diseases and memory disorders.

12. Compound according to any one of Claims 1 to 6, for the treatment of ischemic and tumoral pathologies in which aminopeptidase A is involved.

## Patentansprüche

1. Verbindung, die **dadurch gekennzeichnet ist, dass** sie der Formel (1) entspricht: 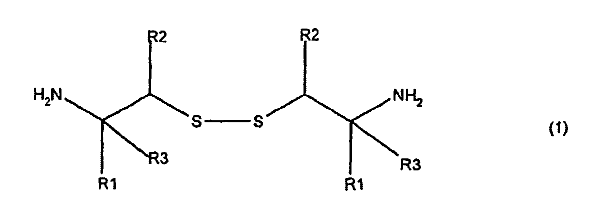 in welcher
• jede Gruppierung R¹ identisch ist mit der anderen Gruppierung R¹ und darstellt:
- eine C₁ bis C₆ Alkylgruppe, C₂ bis C₆ Alkenylgruppe oder C₂ bis C₆ Alkinylgruppe,
- eine (CH₂)ₙ-Benzylgruppe, in welcher n gleich 0 oder 1 ist,
- eine (CH₂)ₘ-(C₃ bis C₆ Cycloalkyl) gruppe, in welcher m gleich 0 oder 1 ist,
wobei jede der Alkyl-, Alkenyl-, Alkinyl-, Benzyl- oder Cycloalkylgruppen mit einer oder zwei durch die Gruppierung A dargestellten Gruppe(n) substituiert ist;
• die Gruppierung A darstellt:
- eine Carboxylatgruppe COOH oder COOR, wobei R eine C₁ bis C₆ Alkylgruppe oder CH₂-Phenylgruppe darstellt;
- eine Sulfonatgruppe SO₃H oder SO₃R', wobei R' eine C₁ bis C₆ Alkylgruppe oder CH₂-Phenylgruppe darstellt;
- eine Phosphonatgruppe PO₃H₂ oder PO₃R₂"R"',
wobei R" und R"' unabhängig voneinander H, eine C₁ bis C₆ Alkylgruppe oder eine CH₂-Phenylgruppe darstellen;
• jede Gruppierung R² identisch ist mit der anderen Gruppierung R² und eine C₁ bis C₆ Alkylgruppe, C₂ bis C₆ Alkenylgruppe oder C₂ bis C₆ Alkinylgruppe darstellt,
wobei jede Alkyl-, Alkenyl- oder Alkinylgruppe frei oder mit der Gruppierung B substituiert ist;
• die Gruppierung B darstellt:
- eine Carboxylatgruppe COOH oder COOR', wobei R' eine C₁ bis C₆ Alkylgruppe oder CH₂-Phenylgruppe darstellt;
- eine freie oder mit einem oder mehreren Radikalen, ausgewählt aus einem Halogenatom, einem eventuell geschützten Hydroxylradikal, einer C₁ bis C₄ Alkylgruppe, einer Cyanogruppe, einer freien, versalzten, veresterten Carboxylgruppe oder einer Amidgruppe, substituierte Phenylgruppe;
• jede Gruppierung R³ identisch ist mit der anderen Gruppierung R³ und ein Wasserstoffatom darstellt.

2. Verbindung nach Anspruch 1, **dadurch gekennzeichnet, dass** R¹ ausgewählt ist unter C₁ bis C₆ Alkylgruppen, C₂ bis C₆ Alkenylgruppen und Benzylgruppen, wobei jede dieser Gruppen mit einer oder zwei durch die in Anspruch 1 definierte Gruppierung A dargestellten Gruppe(n) substituiert ist.

3. Verbindung nach Anspruch 1 oder 2, **dadurch gekennzeichnet, dass** R² ausgewählt ist unter einer C₁ bis C₆ Alkylgruppe oder einer C₂ bis C₆ Alkenylgruppe, wobei jede dieser Gruppen mit einer oder zwei durch die in Anspruch 1 definierte Gruppierung B dargestellten Gruppe(n) substituiert sein kann.

4. Verbindung nach einem der Ansprüche 1 bis 3, **dadurch gekennzeichnet, dass** R¹ eine mit einer Sulfongruppe, einer Phosphongruppe oder einer freien, versalzten oder veresterten Carboxylgruppe substituierte Ethylgruppe darstellt und R² eine mit einer freien oder substituierten Phenylgruppe substituierte Ethylgruppe darstellt.

5. Verbindung nach einem der Ansprüche 1 bis 4, **dadurch gekennzeichnet, dass** es sich um 4,4'-dithiobis-(3,3'-amino-6,6'-phenyl-1-1'-hexansulfonsäure) handelt.

6. Verbindung nach Anspruch 5, **dadurch gekennzeichnet, dass** es sich um 4(S),4'(S),3(S),3'(S)-4'-dithiobis-(3,3'-amino-6,6'-phenyl-1-1'-hexansulfonsäure) handelt.

7. Verbindung nach einem der Ansprüche 1 bis 6, **dadurch gekennzeichnet, dass** sie zur Verwendung in der Therapeutik bestimmt ist.

8. Pharmazeutische Zusammensetzung, **dadurch gekennzeichnet, dass** sie eine Verbindung nach einem der Ansprüche 1 bis 6 enthält.

9. Verwendung einer Verbindung nach einem der Ansprüche 1 bis 6 zur Anfertigung eines Medikaments als selektiver Inhibitor von Aminopeptidase A.

10. Verbindung nach einem der Ansprüche 1 bis 6, zur Behandlung arterieller Hypertonie und direkt oder indirekt damit verbundener Erkrankungen.

11. Verbindung nach einem der Ansprüche 1 bis 6, zur Behandlung einer Erkrankung, ausgewählt aus primärer und sekundärer arterieller Hypertonie, Iktus, myokardialer Ischämie, Herzinsuffizienz und Niereninsuffizienz, Herzinfarkt, einer peripheren Gefäßerkrankung, diabetischer Proteinurie, X-Syndrom, Glaukom, neurodegenerativer Erkrankungen und Gedächtnisstörungen.

12. Verbindung nach einem der Ansprüche 1 bis 6, zur Behandlung ischämischer und Geschwulst-Pathologien, bei welchen Aminopeptidase A eine Rolle spielt.
